(19) Europäisches Patentamt
European Patent Office
Office européen des brevets

(11) Veröffentlichungsnummer: **0 354 495**
**A1**

(12) **EUROPÄISCHE PATENTANMELDUNG**

(21) Anmeldenummer: 89114477.6

(22) Anmeldetag: 04.08.89

(51) Int. Cl.4: C07D 213/69 , A61K 31/44 , C07D 401/06 , C07D 401/14 , A61K 31/495

(30) Priorität: 06.08.88 DE 3826846

(43) Veröffentlichungstag der Anmeldung: 14.02.90 Patentblatt 90/07

(84) Benannte Vertragsstaaten: ES GR

(71) Anmelder: GÖDECKE AKTIENGESELLSCHAFT
Salzufer 16
D-1000 Berlin 10(DE)

(72) Erfinder: Aranda, Julian, Dr.
Sulzgasse 5
D-7801 Vörstetten(DE)
Erfinder: Hartenstein, Johannes, Dr.
Fohrenbühl 23
D-7801 Stegen-Wittental(DE)
Erfinder: Reck, Reinhard, Dr.
Vogesenstrasse 15
D-7831 Sexau(DE)
Erfinder: Schächtele, Christoph, Dr.
Darriwald 16
D-7800 Freiburg(DE)
Erfinder: Rudolph, Claus, Dr.
Riedmattenstrasse 11
D-7801 Vörstetten(DE)
Erfinder: Osswald, Hartmut, Prof. Dr.
Kiefernweg 1
D-7808 Waldkirch 2(DE)
Erfinder: Weinheimer, Günter, Dr.
Sachsenstrasse 4
D-7819 Denzlingen(DE)

(74) Vertreter: Mansmann, Ivo
c/o Gödecke AG - Patentabteilung Postfach 569 - Mooswaldallee 1-9
D-7800 Freiburg(DE)

(54) **Alkoxy-4 (1H)-pyridon-Derivate, Verfahren zu deren Herstellung und deren Verwendung als Arzneimittel.**

(57) Es werden Alkoxy-4(1H)-pyridon-Derivate der allgemeinen Formel I:

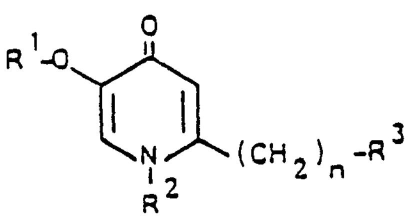

(I)

beschrieben, sowie Verfahren zu deren Herstellung, diese enthaltende Arzneimittel und deren Verwendung zu Prävention und/oder Behandlung von Herz- und Gefäßkrankheiten wie Thrombosen, Arteriosklerose, Hypertonien, von Entzündungprozessen, Allergien, Krebs und bestimmten degenerativen Schäden des Zentralnervensystems.

EP 0 354 495 A1

## Alkoxy-4(1H)-pyridon-Derivate, Verfahren zu deren Herstellung und deren Verwendung als Arzneimittel

Die Erfindung betrifft neue Alkoxy-4(1H)-pyridon-Derivate der allgemeinen Formel I

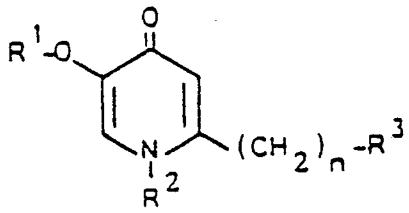

(I)

in welcher

$R^1$ eine geradkettige oder verzweigte, gesättigte oder ungesättigte Alkyl- oder Adamantylalkylgruppe mit jeweils bis zu 22 Kohlenstoffatomen darstellt,

$R^2$ eine geradkettige oder verzweigte, gesättigte oder ungesättigte Alkylgruppe mit bis zu 5 Kohlenstoffatomen, eine Cycloalkylmethylgruppe mit 5 bis 7 C-Atomen im Cycloalkylring, eine unsubstituierte oder durch Halogen, Hydroxy, Alkyl mit bis zu 5 C-Atomen, Alkoxy mit bis zu 5 C-Atomen, Dialkylamino mit bis zu 5 C-Atomen oder Benzyloxy substituierte Phenyl- oder Phenylalkylgruppe mit einem geradkettigen oder verzweigten Alkylrest mit bis zu 5 C-Atomen oder eine Aminoalkylgruppe der allgemeinen Formel II darstellt

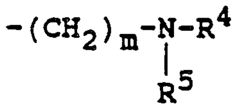

(II)

worin $R^4$ und $R^5$ gleich oder verschieden sind und Wasserstoff oder eine Alkylgruppe mit 1 bis 3 C-Atomen und m eine ganze Zahl von 2 bis 5 bedeuten,

n eine ganze Zahl von 1 bis 5 bedeutet, und

$R^3$ entweder

a) ein Halogenatom, eine Hydroxy-, Cyano-, Carboxamid-oder Alkoxycarbonylgruppe mit bis zu 5 C-Atomen in der Alkylgruppe bedeutet, oder einen Aminorest der allgemeinen Formel III

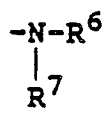

(III)

bedeutet, worin $R^6$ und $R^7$ gleich oder verschieden sind und Wasserstoff, eine Alkyl- oder omega-Hydroxyalkylgruppe mit bis zu 5 C-Atomen, eine unsubstituierte oder durch Halogen, Hydroxy, Alkyl mit bis zu 5 C-Atomen, Alkoxy mit bis zu 5 C-Atomen, Dialkylamino mit bis zu 5 C-Atomen oder Benzyloxy substituierte Phenyl- oder Phenylalkylgruppe mit einem geradkettigen oder verzweigten Alkylrest mit bis zu 5 C-Atomen bedeuten, oder

b) einen Aminorest der allgemeinen Formel IV

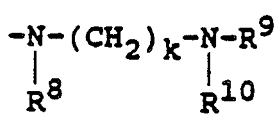

(IV)

darstellt, in welcher $R^8$ Wasserstoff oder eine Alkylgruppe mit bis zu 5 C-Atomen und $R^9$ und $R^{10}$ gleich oder verschieden sind und Wasserstoff, eine Alkylgruppe mit bis zu 5 C-Atomen, eine unsubstituierte oder durch Halogen, Hydroxy, Alkyl mit bis zu 5 C-Atomen, Alkoxy mit bis zu 5 C-Atomen, Dialkylamino mit bis zu 5 C-Atomen oder Benzyloxy substituierte Phenyl- oder Phenylalkylgruppe mit einem geradkettigen oder

verzweigten Alkylrest mit bis zu 5 C-Atomen bedeuten und k eine ganze Zahl von 2 bis 5 bedeutet, oder

c) einen Heterocyclus der allgemeinen Formel V

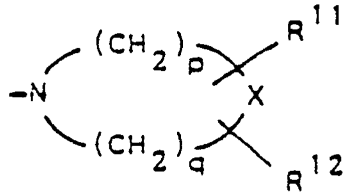

(V)

bedeutet, in welcher $R^{11}$ und $R^{12}$ gleich oder verschieden sind und Wasserstoff, eine Alkylgruppe mit bis zu 3 C-Atomen oder eine Phenyl- oder Phenylalkylgruppe mit einem geradkettigen oder verzweigten Alkylrest mit bis zu 5 C-Atomen bedeuten, X für Sauerstoff, einen Aminorest der allgemeinen Formel VI,

$-N-R^{13}$ (VI)

wobei $R^{13}$ Wasserstoff oder eine Phenyl-, Phenylalkyl-oder Diphenylalkylgruppe mit einem geradkettigen oder verzweigten Alkylrest mit bis zu 5 C-Atomen, wobei im Phenylring bis zu 3 CH-Gruppen durch Stickstoffatome ersetzt sein können, bedeutet, oder für einen Rest der allgemeinen Formel VII

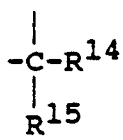

(VII)

steht, worin $R^{14}$ Wasserstoff oder eine unsubstituierte oder durch Halogen, Hydroxy, Alkyl mit bis zu 5 C-Atomen, Alkoxy mit bis zu 5 C-Atomen, Dialkylamino mit bis zu 5 C-Atomen oder Benzyloxy substituierte Phenylgruppe bedeutet, $R^{15}$ Wasserstoff, eine Hydroxy-, Hydroxymethyl-, Cyano-, Aminomethyl-, Carboxamid-, Ethoxy- oder Methoxycarbonylgruppe und p und q gleich oder verschieden sein können und 2 oder 3 bedeuten, oder

d) für einen Rest der allgemeinen Formel VIII

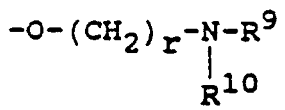

(VIII)

steht, worin $R^9$ und $R^{10}$ die oben genannte Bedeutung haben, und r eine ganze Zahl von 2 bis 5 bedeutet, sowie gegebenenfalls deren pharmakologisch unbedenkliche Salze.

Bevorzugt werden Alkoxy-4(1H)-pyridon-Derivate der allgemeinen Formel I, in welcher

$R^1$ eine geradkettige oder verzweigte, gesättigte oder ungesättigte Alkylgruppe mit 10 bis zu 22 Kohlenstoffatomen oder eine Adamantylethylgruppe, und

$R^2$ einen Methyl-, Ethyl-, Propyl-, Cyclohexylmethylrest, einen unsubstituierten oder durch Halogen, Hydroxy, Methyl, Benzyloxy, Methoxy oder Dimethylamino monosubstituierten Phenyl-, Benzyl- oder Phenylethylrest oder eine Aminoalkylgruppe der allgemeinen Formel II, worin $R^4$ und $R^5$ eine Methylgruppe und m 2 bis 4 bedeuten,

n eine ganze Zahl von 1 bis 3 bedeutet, und

$R^3$ entweder,

a) wenn n gleich 1 ist, einen Rest der allgemeinen Formel III darstellt, in welcher $R^6$ und $R^7$ gleich oder verschieden sind und Wasserstoff, eine Methyl-, omega-Hydroxypropyl- oder Benzylgruppe bedeuten, oder

b) einen Aminorest der allgemeinen Formel IV darstellt, in welcher $R^8$ Wasserstoff oder eine Methylgruppe bedeutet, $R^9$ und $R^{10}$ gleich oder verschieden sind und Wasserstoff, eine Methyl-, Benzyl- oder Phenylethylgruppe und k, 2, 3 oder 4 bedeuten, oder

c) einen Heterocyclus der allgemeinen Formel V darstellt, in welcher $R^{11}$ und $R^{12}$ gleich oder verschieden sind und Wasserstoff oder eine Methylgruppe bedeuten, X für einen Aminorest der allgemeinen Formel VI steht, wobei $R^{13}$ Wasserstoff oder eine Phenyl-, Benzyl-, Pyrimidinyl-, Diphenylmethyl- oder Phenylethylgruppe bedeutet, oder X für einen Rest der allgemeinen Formel VII steht, worin $R^{14}$ für eine Phenylgruppe, $R^{15}$ eine Cyano- oder Aminomethylgruppe bedeutet, und p und q gleich 2 sind.

Insbesondere werden Verbindungen der allgemeinen Formel I, bevorzugt, worin $R^3$ einen Heterocyclus der folgenden Strukturen Va, Vb, Vc, Vd oder Ve darstellt:

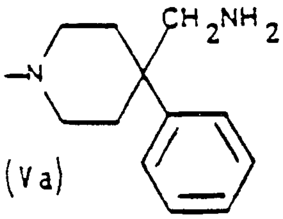
(Va)
,
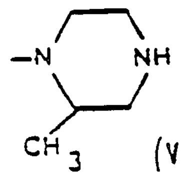
(Vb)
,

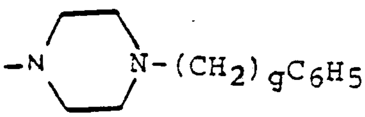
(Vc)

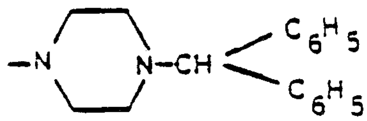
(Vd)
oder

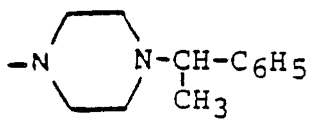
(Ve)

wobei g in Vc 0, 1 oder 2 ist.

Bevorzugt werden auch Verbindungen der allgemeinen Formel I, in welcher $R^3$ entweder, wenn n gleich 1 ist, für einen Rest der allgemeinen Formel VIII steht, worin $R^9$ und $R^{10}$ Methylgruppen und r 2 oder 3 bedeuten, oder, wenn n gleich 2 oder 3 ist, eine Amino-, Cyano-, Hydroxy-, Methoxycarbonyl- oder Carboxamidgruppe darstellt.

Die Erfindung betrifft auch Verfahren zur Herstellung von Alkoxy-4(1H)-pyridon-Derivaten der allgemeinen Formel I, dadurch gekennzeichnet, daß man entweder:

a) eine Verbindung der allgemeinen Formel IX

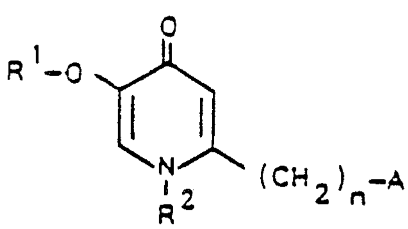
(IX)

in welcher $R^1$, $R^2$ die oben angegebene Bedeutung haben, n gleich 1 ist und A für eine nukleofuge Abgangsgruppe steht, mit Aminoverbindungen der allgemeinen Formel X, XI und XII,

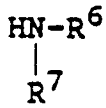 , 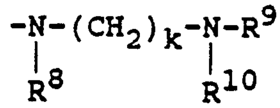 , 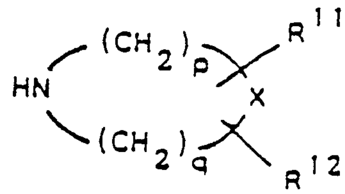

(X) (XI) (XII)

in welcher $R^6$, $R^7$, $R^8$, $R^9$, $R^{10}$, $R^{11}$, $R^{12}$, k, p, q und x die oben angegebene Bedeutung besitzen, in an sich bekannter Weise umsetzt, oder

b) eine Verbindung der allgemeinen Formel XIII,

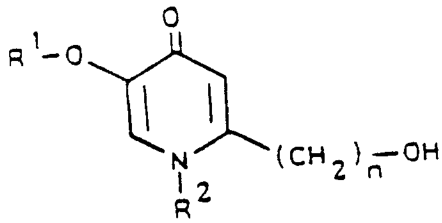

(XIII)

in welcher $R^1$, $R^2$ die oben angegebene Bedeutung haben und n gleich 1 ist, mit einer Verbindung der allgemeinen Formel XIV,

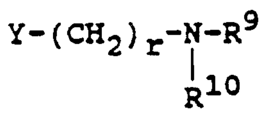

(XIV)

in welcher $R^9$, $R^{10}$ und r die oben genannte Bedeutung haben und Y eine Ausgangsgruppe bedeutet, in einem geeigneten Lösungsmittel unter basischen Bedingungen, umsetzt, oder

c) wenn n gleich 2 oder 3 ist und $R^3$ für eine Amino-, Hydroxy-, Cyano-, Methoxycarbonyl- oder Carboxamidgruppe steht, ein 2-Halogenmethylpyridon der allgemeinen Formel IXa,

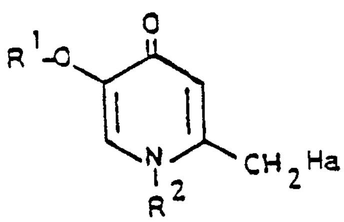

(IXa)

in welcher $R^1$ und $R^2$ die oben angegebene Bedeutung haben, mit einem Cyanessigsäureester nach an sich bekannter Weise umsetzt, verseift und decarboxyliert. Aus den so erhaltenen Cyanoverbindungen der allgemeinen Formel Ia

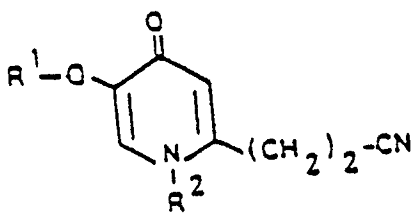

(Ia)

werden dann nach literaturbekannten Methoden die Ester-, Amino-, Hydroxy- und Amidoverbindungen der allgemeinen Formel Ib, Ic, Id und Ie hergestellt. Das folgenden Formelschema veranschaulicht dieses Verfahren.

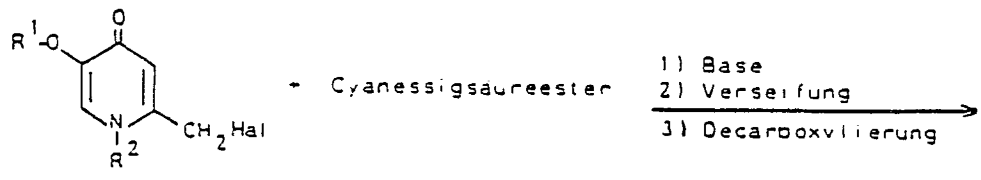


IXa

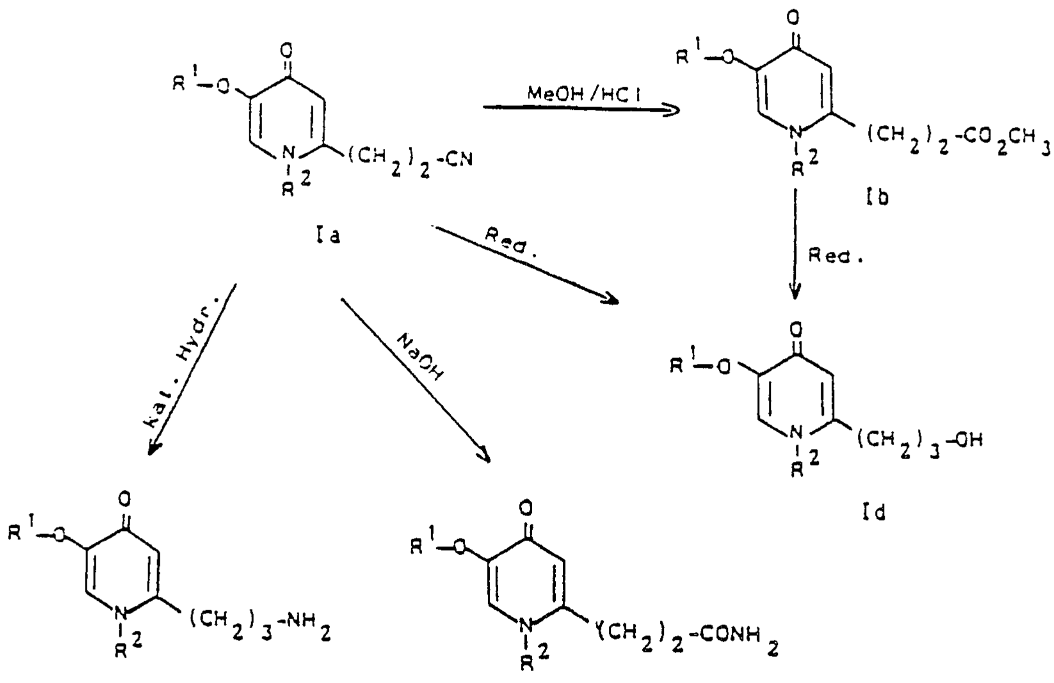


Ia Ib Id Ic Ie

Die Umsetzungen nach Verfahren a) werden in Gegenwart eines inerten Lösungsmittels wie Methanol, Ethanol, Propanol oder 2-Propanol, vorzugsweise Ethanol oder 2-Propanol, bei Temperaturen von 20-90 °C, bevorzugt bei 60-80 °C, durchgeführt. Die Abgangsgruppe A stellt bevorzugt ein Halogenatom, insbesondere Brom dar.

Bei einer zweckmäßigen Ausführungsform zur nukleophilen Substitution des Restes A in IX durch die verwendeten Aminoverbindungen der Formel X, XI und XII wird die Umsetzung in Gegenwart einer Base als säurebindendes Mittel durchgeführt; bevorzugt wird Triethylamin oder Kaliumcarbonat eingesetzt. Gegebenenfalls ist es zweckmäßig, das zur Umsetzung verwendete Aminoderivat in überschüssiger Menge gleichzeitig als säurebindendes Mittel zu verwenden. Die Reaktionszeiten bewegen sich zwischen 1 und 8 Stunden, meist liegen sie bei 2 bis 3 Stunden. Eine Reinigung der erhaltenen Produkte erfolgt in üblicher Weise, beispielsweise durch Umkristallisieren aus einem Lösungsmittel, Überführen in ein Säureadditionssalz oder Säulenchromatographie.

Für den Fall, daß in der allgemeinen Formel I $R^3$ ein Heterocyclus der Formel Va ist,

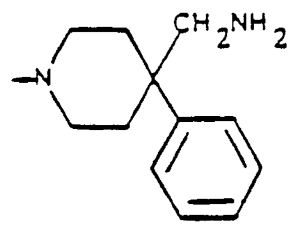

(Va)

wird zuerst eine Verbindung der allgemeinen Formel IX mit 4-Cyano-4-phenyl-piperidin (XIIa) umgesetzt und danach die Cyanogruppe zum Aminomethyl mit Raney-Nickel und Wasserstoff reduziert. Die Reaktion erfolgt zweckmäßig im Autoklaven mit Methanol, mit Ammoniak gesättigt, bei einer Temperatur zwischen 40-100° C, bevorzugt bei 60° C, und einem Druck von 40-120 bar., bevorzugt bei 60 bar.

Im Fall, daß in der allgemeinen Formel I $R^3$ ein Heterocyclus der Formel Vb ist,

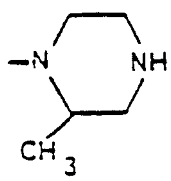

(Vb)

wird eine Verbindung der allgemeinen Formel IX mit einem Piperazinderivat der allgemeinen Formel XIIb,

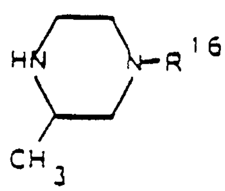

(XIIb)

in welcher $R^{16}$ eine Schutzgruppe bedeutet, beispielsweise eine Ethoxy-, Methoxy-, tert.-Butoxy- oder Benzyloxycarbonylgruppe, bevorzugt eine Methoxycarbonylgruppe, umgesetzt und anschließend die Schutzgruppe abgespalten. Die Abspaltung der Schutzgruppe geschieht nach an sich üblichen Methoden unter sauren Bedingungen. Vorzugsweise wird die Abspaltung der Schutzgruppe $R^{16}$ mit Bromwasserstoff in Eisessig in einem geschlossenen Gefäß unter Normaldruck bei Temperaturen zwischen 5 und 25° C, bevorzugt bei 20° C, durchgeführt.

Bei der Verfahrensvariante b) erfolgt die Durchführung der Reaktion bevorzugt in einem polaren, aprotischen Lösungsmittel wie z.B. Dimethylformamid oder Dimethylsulfoxid, in Gegenwart einer geeigneten Base, vorzugsweise Natriumhydrid (vgl. Eur. Pat. Appl. EP 171,814). Die Umsetzungen werden bei Temperaturen von 20-100° C, zweckmäßig bei Temperaturen von 50-60° C, durchgeführt. Eine Reinigung der erhaltenen Produkte erfolgt wiederum durch Umkristallisieren aus einem Lösungsmittel, Überführen in ein Säureadditionssalz oder Säulenchromatographie.

Die austretende Gruppe Y der Ausgangsverbindung der allgemeinen Formel XIV stellt ein Halogenatom, vorzugsweise Chlor oder Brom, insbesondere Chlor, dar. Weiterhin kommen beispielsweise als Abgangsgruppe aromatische oder aliphatische Sulfonsäurereste in Betracht, wie der p-Toluolsulfonsäure- oder der Methansulfonsäurerest.

Bei der Verfahrensvariante c) werden die Verbindungen der allgemeinen Formel Ia hergestellt, indem man ein 2-Brommethylpyridon-Derivat der allgemeinen Formel IXa, worin $R^1$ und $R^2$ die angegebene Bedeutung haben, mit Cyanessigsäure-tert.-butylester umsetzt. Zweckmäßigerweise wird die Reaktion in einem inerten, polaren aprotischen Lösungsmittel, bevorzugt Dimethylformamid, in Gegenwart einer Base, bevorzugt Kalium-tert.-butylat, bei Temperaturen von 20-100° C, bevorzugt bei 50-60° C, durchgeführt. Die so erhaltenen Zwischenprodukte werden einer sauren oder alkalischen Hydrolyse unterworfen. Vorzugsweise wird alkalisch hydrolysiert, beispielsweise bei Zimmertemperatur in wäßriger Kali- oder Natronlauge und Methanol. In den erhaltenen Verbindungen wird nun die Carbonsäuregruppe nach an sich bekannten Methoden, z.B. durch Schmelzen oder Erhitzen in einem hochsiedenden Lösungsmittel, abgespalten.

Durch katalytische Hydrierung der Cyanogruppe in den Verbindungen der allgemeinen Formel Ia

werden die Verbindungen der allgemeinen Formel Ib hergestellt. Die Reaktion erfolgt zweckmäßig im Autoklaven mit methanolischem Ammoniak und Raney-Nickel als Katalysator bei Temperaturen zwischen 40-100 ° C, bevorzugt bei 60 ° C und einem Druck von 40-120 bar, bevorzugt bei 60 bar.

Für den Fall, daß bei den Verbindungen der allgemeinen Formel I $R^3$ eine Methoxycarbonylgruppe bedeutet und n gleich 2 ist, läßt man die entsprechenden Cyanoverbindungen der allgemeinen Formel Ia mit methanolischem Chlorwasserstoff reagieren. Vorzugsweise wird die Reaktion in einem geschlossenen Gefäß unter Normaldruck bei Zimmertemperatur und nachfolgender Hydrolyse durchgeführt. Durch Umsetzen einer Verbindung der allgemeinen Formel Ia mit Natriumborhydrid in siedendem 2-Propanol und nachfolgender Behandlung mit Natriumhydroxidlösung werden die Alkoxy-4(1H)-pyridon-Derivate der allgemeinen Formel Id und Ie erhalten (vgl. R.A. Egli, Helv. Chim. Acta 53, 47 (1970)).

Die Verbindungen der allgemeinen Formel Ia-Ie können beispielsweise durch Säulenchromatographie, Überführen in eine Säure und/oder Umkristallisieren aus einem Lösungsmittel gereinigt werden. Gegebenenfalls können die Verbindungen der allgemeinen Formel Ia in an sich bekannter Weise, durch Hydrolyse mit wäßrigen oder alkoholischen Alkalimetallhydroxiden, beispielsweise Kaliumhydroxid oder Natriumhydroxid, bei Raumtemperatur in die Verbindungen der allgemeinen Formel Ie umgewandelt werden.

Gegebenenfalls können die Verbindungen der Formel Ib durch Reduktion des Carbonsäureesterrestes in die Verbindungen der allgemeinen Formel Id umgewandelt werden. Die Reduktion kann nach allgemein bekannten Methoden durchgeführt werden, z.B.mit komplexen Methallhydriden wie Natriumborhydrid in tert.-Butanol/Methanol (vgl. K. Soai, Synthetic Communications 12, 463 (1982)).

Die als Zwischenprodukte eingesetzten 4(1H)-Pyridon-Derivate der allgemeinen Formel IX und XIII, in welcher R' und $R^2$ die angegebene Bedeutung haben, n gleich 1 ist und A für ein Bromatom steht, werden aus Kojisäure nach literaturbekannten Verfahren, wie das folgende Reaktionsschema zeigt, hergestellt:

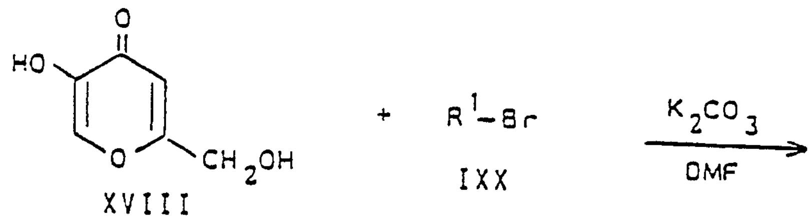


XVIII + IXX

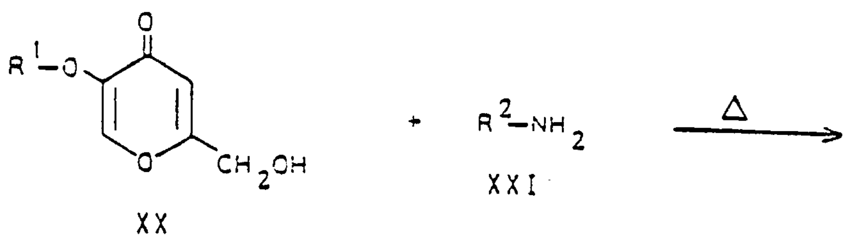


XX + XXI

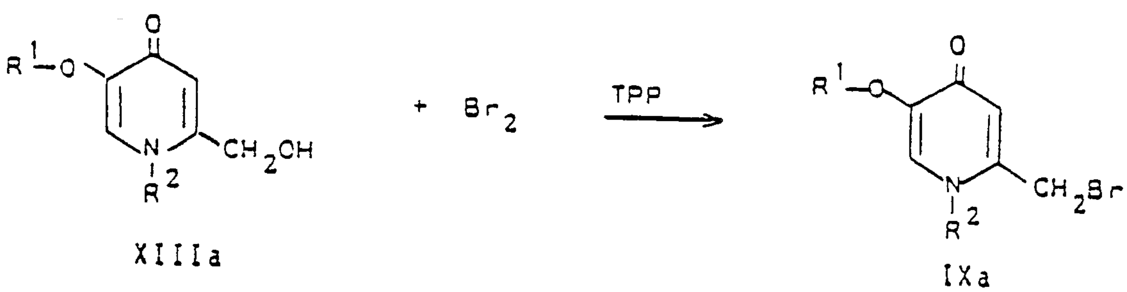


XIIIa + $Br_2$ → IXa

Dabei wird Kojisäure (XVIII) mit dem entsprechenden Alkylbromid IXX nach üblichen, in der Literatur beschriebenen Verfahren (z.B. US 4644-071-A; Eur. Pat. Appl. EP 171,814; A.F. Thomas und A. Marxer, Helv. Chim. Acta 43, 469 (1960); Eur. Pat. Appl. EP 0209751) eingesetzt. Bevorzugt wird diese Reaktion mit Kaliumcarbonat als Base und Dimethylformamid als Lösungsmittel durchgeführt.

Die 2-Hydroxymethyl-4(1H)-pyridon-Derivate der allgemeinen Formel XIIIa werden hergestellt, indem man nach an sich bekannter Weise (vgl. z.B. Counsell et al., J. Med. Chem. 17(1), 1-5 (1974); J.H. Looker und M.D. Cliffton, J. Heterocyclic Chem. 23, 5 (1986); Tsutomu Teitei, Austr. J. Chem. 36, 2307-15 (1983); Can. 978,958; S. Hünig und G. Köbrich, Liebigs Ann. Chem. 609, 181 (1958); K. Imafuku et al., Bull. chem. Soc. Japan 52, 107 (1979)) ein Pyranon-Derivat der allgemeinen Formel XX mit einem Amin der allgemeinen Formel XXI bei Temperaturen von 80-120 °C, bevorzugt bei 100 °C, reagieren läßt. Bei Aminen mit niedrigem Siedepunkt wird die Reaktion im Autoklaven in Gegenwart eines inerten, polaren Lösungsmittels, beispielsweise Ethanol, durchgeführt. Die Produkte werden durch Säulenchromatographie oder Kristallisation gereinigt.

Für die Überführung der Hydroxymethylgruppe in Verbindungen der Formel XIII in eine Brommethylgruppe eignet sich besonders das aus Brom und Triphenylphosphin gebildete Triphenylphosphindibromid (vgl. Fieser and Fieser, Reagents for Organic Synthesis, Wiley-Interscience, 1975, Vol. 5, p. 729). Die Durchführung der Reaktion erfolgt, indem man eine Lösung oder Suspension des in situ hergestellten Triphenylphosphindibromids in einem geeigneten, wasserfreien Lösungsmittel, bevorzugt Dichlormethan oder Toluol, mit der betreffenden 2-Hydroxymethyl-4(1H)-pyridon Verbindung der Formel XIII umsetzt, im Temperaturbereich von 30-100 °C, vorzugsweise jedoch bei 50 °C, wenn Dichlormethan oder bei 70 °C, wenn Toluol das Lösungsmittel ist. Die 2-Brommethylverbindungen der Formel IXa werden nach der

Reaktion als Hydrobromide isoliert und durch Kristallisation gereinigt oder ohne Reinigung weiter umgesetzt.

Sofern die erfindungsgemäßen Verbindungen der Formel I ein chirales Zentrum aufweisen, können sie entweder als racemische Gemische oder in Form der Enantiomeren vorliegen. Racemische Gemische lassen sich mit den üblichen Methoden in die Enantiomeren trennen.

Da die Verbindungen der allgemeinen Formel I basische Zentren besitzen, überführt man sie zum Zwecke der Reinigung und/oder aus galenischen Gründen mit Hilfe von anorganischen und organischen Säuren bevorzugt in kristalline, pharmakologisch verträgliche Salze. Als Säuren kommen z.B. Salzsäure, Schwefelsäure, Phosphorsäure, Bromwasserstoffsäure, Essigsäure, Weinsäure, Milchsäure, Zitronensäure, Äpfelsäure, Salicylsäure, Ascorbinsäure, Malonsäure, Fumarsäure, Oxalsäure oder Bernsteinsäure in Frage. Die Säureadditionssalze werden in der Regel in an sich bekannter Weise durch Mischen der freien Base oder deren Lösungen mit der entsprechenden Säure oder deren Lösungen in einem organischen Lösunsgsmittel, beispielsweise einem niederen Alkohol wie Methanol, Ethanol oder 2-Propanol oder einem niederen Keton wie Aceton oder 2-Butanon oder einem Ether wie Diethylether, Diisopropylether, Tetrahydrofuran oder Dioxan, erhalten.

Die erfindungsgemäßen Verbindungen der allgemeinen Formel I weisen interessante pharmakologische Eigenschaften auf. Insbesondere hemmen sie die Proteinkinase C, ein Calcium- und Phospholipid-abhängiges Schlüsselenzym, das bei der intrazellulären Signalkettentransduktion eine entscheidende Rolle spielt (Y. Nishizuka, Science 233, 305-312 (1986)) und eng mit der Regulation von kontraktilen, sekretorischen und proliferativen Prozessen verknüpft ist.

Aufgrund dieser Eigenschaften können die erfindungsgemäßen Verbindungen zur Prävention und/oder Behandlung von Herz- und Gefäßkrankheiten wie Thrombosen, Arteriosklerose, Hypertonien, von Entzündungsprozessen, Allergien, Krebs und bestimmten degenerativen Schäden des Zentralnervensystems verwendet werden. Ein weiterer Gegenstand der vorliegenden Erfindung ist daher die Verwendung von Alkoxy-4(1H)-pyridon-Derivaten der allgemeinen Formel I bei der Behandlung von Herz- und Gefäßkrankheiten, Entzündungsprozessen, Allergien, Krebs und Erkrankungen des Zentralnervensystems.

Überraschenderweise wurde bei den erfindungsgemäßen Verbindungen außerdem gefunden, daß sie eine endothelabhängige Relaxation der Gefäßmuskulatur bewirken.

Die erfindungsgemäßen Verbindungen der allgemeinen Formel I können in flüssiger oder fester Form oral oder parenteral appliziert werden. Als Injektionslösung kommt vor allem Wasser zur Anwendung, welches die bei Injektionslösungen üblichen Zusätze wie Stabilisierungsmittel, Lösungsvermittler oder Puffer enthält

Derartige Zusätze sind z. B. Tartrat- und Citrat-Puffer, Ethanol, Komplexbildner (wie Äthylendiamintetraessigsäure und deren nicht-toxische Salze) sowie hochmolekulare Polymere (wie flüssiges Polyäthylenoxid) zur Viskositätsregulierung. Feste Trägerstoffe sind z. B. Stärke, Lactose, Mannit, Methylcellulose, Talkum, hochdisperse Kieselsäuren, höhermolekulare Fettsäuren (wie Stearinsäure), Gelatine, Agar-Agar, Calciumphosphat Magnesiumstearat, tierische und pflanzliche Fette, feste hochmolekulare Polymere (wie Polyäthylenglykol); für orale Applikation geeignete Zubereitungen können gewünschtenfalls zusätzliche Geschmacks-und/oder Süßstoffe enthalten. Die enteral oder parenteral verabreichten Einzeldosen liegen im Bereich von 0,5 bis 1000 mg, vorzugsweise in Dosen von 1 bis 100 mg.

Die vorliegende Erfindung wird durch die nachfolgenden Ausführungsbeispiele näher erläutert.

Beispiel 1

2-Hydroxymethyl-5-octadecyloxy-4-pyranon

Eine Mischung aus 56,8 g (0,4 Mol) Kojisäure, 55,3 g (0,4 Mol) $K_2CO_3$, 133,3 g (0,4 Mol) 1-Octadecylbromid, 1 g Kaliumjodid und 450 ml absoluten Dimethylformamid wird 10 Stunden bei 90 °C gerührt. Nach dem Abkühlen wird die Mischung mit 1,5 Liter Wasser versetzt, der sich gebildete Niederschlag abfiltriert, einmal mit Ethylacetat gut verrührt, abfiltriert und aus 2-Propanol unter Zusatz von Aktivkohle umkristallisiert. Man erhält ein fast farbloses Produkt vom Schmp. 71-73 °C.

In analoger Weise, wie in Beispiel 1 beschrieben, erhält man:

Beispiel Nr.

2 -2-Hydroxymethyl-5-tetradecyloxy-4-pyranon, Schmp. 65-68 °C aus 2-Propanol;

3 5-Eicosanyloxy-2-hydroxymethyl-4-pyranon, Schmp. 81-84 °C aus 2-Propanol;

Beispiel 4

5-Butyloxy-2-hydroxymethyl-4-pyranon

Zu einem Gemisch von 50 g (0,352 Mol) Kojisäure, 48,7 g (0,352 Mol) $K_2CO_3$, 1 g Kaliumjodid und 420 ml absoluten Dimethylformamid werden unter kräftigen Rühren bei Raumtemperatur 48,3 (0,352 Mol) 1-Butylbromid zugetropft. Danach wird die Reaktionsmischung 3 Stunden bei 90°C gerührt. Nach dem Abkühlen wird das Lösungsmittel im Vakuum abgezogen, der Rückstand mit Wasser versetzt und mit Dichlormethan extrahiert.

Die organische Phase wird abgetrennt, getrocknet, das Lösungsmittel abgedampft und der Rückstand aus Ethylacetat umkristallisiert. Man erhält ein fast farbloses Produkt vom Schmp. 68-73°C.

In analoger Weise, wie in Beispiel 4 beschrieben, erhält man:

Beispiel Nr.

5 5-Decyloxy-2-hydroxymethyl-4-pyranon, Schmp. 46-50°C aus Ethylacetat;

Beispiel 6

2-Hydroxymethyl-5-(9-cis-octadecenyloxy)-4-pyranon

Eine Mischung aus 14,2 g (0,1 Mol) Kojisäure, 13,8 g (0,1 Mol) $K_2CO_3$, 1 g Kaliumjodid, 33,14 g (0,1 Mol) 1-Brom-9-cis-octadecen und 120 ml absolutes Dimethylformamid wird 3 Stunden bei 90°C gerührt. Nach dem Abkühlen wird das Lösungsmittel im Vakuum abgezogen, der Rückstand mit Wasser versetzt und mit Dichlormethan extrahiert. Die organische Phase wird abgetrennt, getrocknet und eingedampft. Der Rückstand wird säulenchromatographisch an Kieselgel mit Dichlormethan-Methanol 100:1 gereinigt. Man erhält ein gelbes Öl.

$^1$H-NMR (90 MHz; $CDCl_3$), delta (ppm): 0,85 (t, 3H); 1,30 (s, 22H); 1,7-2,2 (m, 6H); 3,8 (t, 2H); 4,2 (t, 1H); 4,4 (d, 2H); 5,3 (t, 2H); 6.5 (s, 1H); 7,55 (s, 1H).

In analoger Weise, wie in Beispiel 6 beschrieben, erhält man:

Beispiel Nr.

7 2-Hydroxymethyl-5-(2-octyldecyloxy)-4-pyranon, gelbes Öl, IR (KBr): 1640 (C=O);

8 5-[2-(1-Adamantyl)ethoxy]-2-hydroxymethyl-4-pyranon, Schmp. 155-157°C aus 2-Propanol/Wasser;

Beispiel 9

1-Benzyl-2-hydroxymethyl-5-octadecyloxy-4(1H)-pyridon

Eine Mischung aus 50,0 g (0,127 Mol) 2-Hydroxymethyl-5-octadecyloxy-4-pyranon und 70 ml Benzylamin wird 2 Stunden bei 100-110°C gerührt. Nach Abkühlen auf ca. 50°C wird die dunkle Lösung mit 400 ml Wasser versetzt und verrührt. Der so gebildete Niederschlag wird abfiltriert, gewaschen, getrocknet aus 2-Propanol unter Zusatz von Aktivkohle umkristallisiert. Man erhält farblose Kristalle vom Schmp. 120-121°C.

In analoger Weise, wie in Beispiel 9 beschrieben, erhält man:

Beispiel Nr.

10 1-Benzyl-5-butoxy-2-hydroxymethyl-4(1H)-pyridon, Schmp. 123-124°C aus 2-Propanol;

11 1-Benzyl-5-decyloxy-2-hydroxymethyl-4(1H)-pyridon, Schmp. 128-129°C aus 2-Propanol;

12 1-Benzyl-2-hydroxymethyl-5-tetradecyloxy-4(1H)-pyridon, Schmp. 121-123°C aus 2-Propanol;

13 1-Benzyl-5-eicosanyloxy-2-hydroxymethyl-4(1H)-pyridon, Schmp. 120-122°C aus 2-Propanol;

14 1-Benzyl-2-hydroxymethyl-5-(9-cis-octadecenyloxy)-4(1H)-pyridon, Schmp. 92-93°C aus Ethanol;

15 2-Hydroxymethyl-1-(4-methylbenzyl)-5-octadecyloxy-4(1H)-pyridon, Schmp. 122-125°C aus 2-Propanol;

16 1-(4-Chlorbenzyl)-2-hydroxymethyl-5-octadecyloxy-4(1H)-pyridon, Schmp. 125-127°C aus 2-Propanol;

17 2-Hydroxymethyl-1-(4-methoxybenzyl)-5-octadecyloxy-4(1H)-pyridon, Schmp. 120-122°C aus 2-Propanol;

18 2-Hydroxymethyl-1-(2-methoxybenzyl)-5-octadecyloxy-4(1H)-pyridon, Schmp. 107-110 °C aus Diisopropylether/2-Propanol;

19 1-Cyclohexylmethyl-2-hydroxymethyl-5-octadecyloxy-4(1H)-pyridon, Schmp. 126-128 °C aus 2-Propanol;

20 1-(4-Dimethylaminobenzyl)-2-hydroxymethyl-5-octadecyloxy-4(1H)-pyridon, Schmp. 109-114 °C aus Ethanol;

21 1-(3-Dimethylaminopropyl)-2-hydroxymethyl-5-octadecyloxy-4(1H)-pyridon, Schmp. 87-90 °C aus Ethylacetat;

22 1-(4-Benzyloxybenzyl)-2-hydroxymethyl-5-octadecyloxy-4(1H)-pyridon, Schmp. 116-120 °C, mit Ether verrührt;

23 1-Benzyl-2-hydroxymethyl-5-(2-octyldecyloxy)-4(1H)-pyridon, gelbes Öl, IR (KBr): 1630 (C=O);

24 5-[2-(1-Adamantyl)ethoxy]-1-benzyl-2-hydroxymethyl-4(1H)-pyridon, Schmp. 246-248 °C, mit Ether verrührt;

Beispiel 25

(±)-2-Hydroxymethyl-5-octadecyloxy-1-(1-phenylethyl)-4(1H)-pyridon

Eine Mischung aus 8,0 g (0,02 Mol) 2-Hydroxymethyl-5-octadecyloxy-4-pyranon, 4,8 g (0,04 Mol) (±)-1-Phenylethylamin, 1,5 g $Na_2CO_3$, 10 ml Wasser und 100 ml Ethanol wird 72 Stunden unter Rückfluß gekocht. Nach dem Abkühlen wird das Lösungsmittel und überschüssiges Amin im Vakuum abdestilliert, der Rückstand mit Wasser versetzt und mit Dichlormethan extrahiert. Der Extrakt wird über $Na_2SO_4$ getrocknet, eingedampft und das erhaltene Harz zur Reinigung an Kieselgel mit Dichlormethan mit 1-2% Methanol chromatographiert. Man erhält ein gelbes Öl, welches beim Stehen wachsartig wird. IR (KBr): 1630 (C=O), MS (m/l) 497 ($M^+$).

In analoger Weise, wie in Beispiel 25 beschrieben, erhält man:

Beispiel Nr.

26 (-)-2-Hydroxymethyl-5-octadecyloxy-1-(1-phenylethyl)-4(1H)-pyridon, wachsartig, IR (KBr): 1630 (C=O), $[\alpha]_D$ = -15,9° (C = 2.42 Methanol);

27 (+)-2-Hydroxymethyl-5-octadecyloxy-1-(1-phenylethyl)-4(1H)-pyridon, wachsartig, IR (KBr): 1630 (C=O), $[\alpha]_D$ = +16,5° (C = 2.01 Methanol);

Beispiel 28

2-Hydroxymethyl-5-octadecyloxy-1-propyl-4(1H)-pyridon

Eine Mischung aus 20,0 g (51 mMol), 2-Hydroxymethyl-5-octadecyloxy-4-pyranon und 30 ml einer 40%igen Lösungen aus Propylamin in Ethanol wird im Autoklaven bei 100-110 °C 5 Stunden erhitzt. Nach dem Abkühlen wird das gebildete Kristallisat mit Wasser aufgenommen, verrührt, abfiltriert, getrocknet und aus Ethylacetat umkristallisiert. Man erhält farblose Kristalle vom Schmp. 120-121 °C.

In analoger Weise, wie in Beispiel 28 beschrieben, erhält man:

Beispiel Nr.

29 2-Hydroxymethyl-1-methyl-5-octadecyloxy-4(1H)-pyridon, Schmp. 96-99 °C aus 2-Propanol;

Beispiel 30

2-Hydroxy-5-octadecyloxy-1-phenyl-4(1H)-pyridon

5,0 g (12 mMol) 2-Hydroxymethyl-5-octadecyloxy-4-pyranon werden in 90 ml Wasser und 2 ml konz. Salzsäure suspendiert und mit 4,0 g (43 mMol) Anilin versetzt. Die Mischung wird 24 Stunden am Rückfluß erhitzt, abgekühlt, der sich gebildete Niederschlag von der wäßrigen Lösung abgetrennt und aus Ethylacetat

umkristallisiert. Man erhält ein fast farbloses Produkt vom Schmp. 67-72° C.

In analoger Weise, wie in Beispiel 30 beschrieben, erhält man: Beispiel Nr.

31 1-(4-Benzyloxyphenyl)-2-hydroxymethyl-5-octadecyloxy-4(1H)-pyridon, schmp. 68-70° C, roh weiterverarbeitet;

## Beispiel 32

### 1-Benzyl-2-brommethyl-5-octadecyloxy-4(1H)-pyridon . Hydrobromid

Zur Lösung von 26.5 g (0,1 Mol) Triphenylphosphin in 600 ml absol. Toluol werden unter Rühren bei Raumtemperatur 5,1 ml (0,1 Mol) Brom zugetropft, wobei sich ein weißer, kristalliner Niederschlag bildet. Nach 20 Minuten Weiterrühren bei Raumtemperatur werden 48,17 g (0,1 Mol) 1-Benzyl-2-hydroxymethyl-5-octadecyloxy-4(1H)-pyridon in Portionen zugefügt. Danach wird die Reaktionsmischung 2 Stunden bei 70° C gerührt, abgekühlt, das ausgefallene Produkt abfiltriert, mit Toluol gewaschen, getrocknet und aus 2-Propanol umkristallisiert. Man erhält hellbeige Kristalle vom Schmp. 92-95° C.

In analoger Weise, wie in Beispiel 32 beschrieben, erhält man:

Beispiel Nr.

33 1-Benzyl-2-brommethyl-5-butoxy-4(1H)-pyridon . Hydrobromid, Sirupartig, roh weiterverarbeitet;

34 1-Benzyl-2-brommethyl-5-decyloxy-4(1H)-pyridon . Hydrobromid, Sirupartig, roh weiterverarbeitet;

35 1-Benzyl-2-brommethyl-5-tetradecyloxy-4(1H)-pyridon . Hydrobromid, Schmp. 135-137° C, mit Aceton verrührt;

36 1-Benzyl-2-brommethyl-5-eicosanyloxy-4(1H)-pyridon . Hydrobromid, Schmp. 97-100° C, mit Aceton verrührt;

37 1-Benzyl-2-brommethyl-5-(9-cis-octadecenyloxy)-4(1H)-pyridon . Hydrobromid;

38 2-Brommethyl-1-(4-methylbenzyl)-5-octadecyloxy-4(1H)-pyridon . Hydrobromid, Schmp. 106-110° C aus Toluol;

39 2-Brommethyl-1-(4-chlorbenzyl)-5-octadecyloxy-4(1H)-pyridon . Hydrobromid, Schmp. 138-148° C, mit Toluol verrührt;

40 2-Brommethyl-1-(4-methoxybenzyl)-5-octadecyloxy-4(1H)-pyridon . Hydrobromid, Schmp. 94-97° C aus Toluol;

41 2-Brommethyl-1-(2-methoxybenzyl)-5-octadecyloxy-4(1H)-pyridon . Hydrobromid, roh weiterverarbeitet;

42 2-Brommethyl-1-cyclohexylmethyl-5-octadecyloxy-4(1H)-pyridon . Hydrobromid, roh weiterverarbeitet;

43 2-Brommethyl-1-(4-dimethylaminobenzyl)-5-octadecyloxy-4(1H)-pyridon . Hydrobromid, roh weiterverarbeitet;

44 2-Brommethyl-5-octadecyloxy-1-propyl-4(1H)-pyridon . Hydrobromid, Schmp. 85-87° C aus Aceton;

45 2-Brommethyl-1-methyl-5-octadecyloxy-4(1H)-pyridon . Hydrobromid, Schmp. 147-148° C aus Aceton;

46 2-Brommethyl-5-octadecyloxy-1-phenyl-4(1H)-pyridon . Hydrobromid, Schmp. 136-140° C aus 2-Propanol;

47 1-(4-Benzyloxybenzyl)-2-brommethyl-5-octadecyloxy-4(1H)-pyridon . Hydrobromid, roh weiterverarbeitet;

48 1-(4-Benzyloxyphenyl)-2-brommethyl-5-octadecyloxy-4(1H)-pyridon . Hydrobromid, roh weiterverarbeitet;

## Beispiel 49

### 1-Benzyl-2-brommethyl-5-(2-octyldecyloxy)-4(1H)-pyridon • Hydrobromid

Zur Lösung von 1,3 g (5 mMol) Triphenylphosphin in 10 ml Dichlormethan wird unter Rühren bei Raumtemperatur die Lösung von 0,25 ml (5 mMol) Brom in 2,5 ml Dichlormethan zugetropft. Nach weiteren 20 Minuten Rühren wird die Lösung von 2,4 g (5mMol) 2-Hydroxymethyl-5-(2-octyldecyloxy)-4-pyranon in 10 ml Dichlormethan zugetropft. Die Reaktionsmischung wird 4 Stunden unter Rückfluß gekocht. Nach dem

Abkühlen wird das Lösungsmittel abdestilliert, der Rückstand mit Ether verrührt, das gebildete Kristallisat abfiltriert, getrocknet und ohne weitere Reinigung umgesetzt.

In analoger Weise, wie in Beispiel 49 beschrieben, erhält man:

Beispiel Nr.

50 5-[2-(1-Adamantyl)ethoxy]-1-benzyl-2-brommethyl-4(1H)-pyridon • Hydrobromid, Schmp. 200-203 °C aus 2-Propanol;

51 (±)-2-Brommethyl-5-octadecyloxy-1-(1-phenylethyl)-4(1H)-pyridon • Hydrobromid, roh weiterverarbeitet;

52 (-)-2-Brommethyl-5-octadecyloxy-1-(1-phenylethyl)-4(1H)-pyridon • Hydrobromid, roh weiterverarbeitet;

53 (+)-2-Brommethyl-5-octadecyloxy-1-(1-phenylethyl)-4(1H)-pyridon • Hydrobromid, roh weiterverarbeitet;

## Beispiel 54

### 1-Benzyl-2-[N-(3-dimethylaminopropyl)aminomethyl]-5-octadecyloxy-4(1H)-pyridon . Dioxalat

Zur Lösung von 2,5 g (25 mMol) 3-Dimethylamino-1-propylamin in 30 ml 2-Propanol werden unter Rühren bei Raumtemperatur 3,1 g (5 mMol) 1-Benzyl-2-brommethyl-5-octadecyloxy-4(1H)-pyridon . Hydrobromid in Portionen eingetragen. Das erhaltene Gemisch wird 1,5 Stunden bei 60 °C gerührt, abgekühlt, im Vakuum eingedampft, der Rückstand mit Wasser versetzt und mit Dichlormethan extrahiert. Die Dichlormethanlösung wird über $Na_2SO_4$ getrocknet, filtriert und im Vakuum zu einem ölartigen Feststoff eingedampft, dieser wird durch Säulenchromatographie über Kieselgel unter Elution mit Dichlormethan/Methanol, $NH_3$ ges., 9:1, gereinigt und dann in Methanol aufgelöst. Die Lösung wird mit einer Lösung von Oxalsäure in Methanol versetzt, das ausgefallene Salz abgesaugt und aus Methanol/Wasser 2,5:1 umkristallisiert. Man erhält farblose Kristalle vom Schmp. 161-164 °C.

In analoger Weise, wie in Beispiel 54 beschrieben, erhält man:

Beispiel Nr.

44 1-Benzyl-2-[N-(3-hydroxypropyl)aminomethyl]-5-octadecyloxy-4(1H)-pyridon, Schmp. 89-91 °C aus Diisopropylether/2-Propanol;

56 2-[N-(3-Dimethylaminopropyl)aminomethyl]-1-methyl-5-octadecyloxy-4(1H)-pyridon . Trihydrochlorid . 1 2 $H_2O$, Schmp. 230 °C (Zers.) aus Ethanol;

57 1-Benzyl-2-[N-(2-dimethylaminoethyl)-N-methylaminomethyl]-5-octadecyloxy-4(1H)-pyridon . Trihydrochlorid, Schmp. 172-174 °C aus 2-Propanol;

58 2-[N-(3-Hydroxypropyl)aminomethyl]-1-methyl-5-octadecyloxy-4(1H)-pyridon, Schmp. 79-82 °C mit Ether digeriert;

59 2-[N-(3-Dimethylaminopropyl)aminomethyl]-5-octadecyloxy-1-propyl-4(1H)-pyridon . Dioxalat, Schmp. 171-172 °C aus Methanol;

60 2-[N-(3-Aminopropyl)aminomethyl]-1-methyl-5-octadecyloxy-4(1H)-pyridon . Trihydrochlorid, Schmp. 212-215 °C aus Ethanol;

61 2-[N-(3-Aminopropyl)aminomethyl]-1-benzyl-5-octadecyloxy-4(1H)-pyridon . Trihydrochlorid, Schmp. 180-183 °C aus Ethanol;

62 2-[N-(3-Aminopropyl)aminomethyl]-1-propyl-5-octadecyloxy-4(1H)-pyridon . Trihydrochlorid, Schmp. 204-205 °C aus 2-Propanol/Methanol;

63 1-Benzyl-5-butyloxy-2-[N-(3-dimethylaminopropyl)aminomethyl]-4(1H)-pyridon . Dioxalat . 3/4 $H_2O$, Schmp. 179-180 °C aus 2-Propanol;

64 1-Benzyl-5-decyloxy-2-[N-(3-dimethylaminopropyl)aminomethyl]-4(1H)-pyridon . Dioxalat . 1/2 $H_2O$, Schmp. 166-167 °C aus 2-Propanol;

65 1-Benzyl-2-[N-(3-dimethylaminopropyl)aminomethyl]-5-tetradecyloxy-4(1H)-pyridon . Dioxalat . 1 $H_2O$, Schmp. 163-167 °C aus Methanol/Wasser;

66 1-Benzyl-2-[N-(3-dimethylaminopropyl)aminomethyl]-5-eicosanyloxy-4(1H)-pyridon . Dioxalat . 1 $H_2O$, Schmp. 165 °C aus 2-Propanol/$H_2O$;

67 2-[N-(2-Aminoethyl)aminomethyl]-1-benzyl-5-octadecyloxy-4(1H)-pyridon . 1/4 $H_2O$, Schmp. 87-89 °C aus Diisopropylether;

68 1-Cyclohexylmethyl-2-[N-(3-dimethylaminopropyl)aminomethyl]-5-octadecyloxy-4(1H)-pyridon . difumarat, Schmp. 142-145 °C aus Ethanol;

69 1-(4-Chlorbenzyl)-2-[N-(3-dimethylaminopropyl)aminomethyl]-5-octadecyloxy-4(1H)-pyridon . Difumarat . 1.25 $H_2O$, Schmp. 121-123° C aus Ethanol;

70 2-[N-(3-Dimethylaminopropyl)aminomethyl]-5-octadecyloxy-1-phenyl-4(1H)-pyridon, Schmp. 50-52° C aus Diisopropylether;

71 1-Benzyl-2-[N-(3-N-benzyl-N-methylaminopropyl)aminomethyl]-5-octadecyloxy-4(1H)-pyridon . Difumarat, Schmp. 140-142° C aus Ethanol;

72 2-[N-(3-Dimethylaminopropyl)aminomethyl]-1-(4-methoxybenzyl)-5-octadecyloxy-4(1H)-pyridon . Trihydrochlorid . $H_2O$, Schmp. 140-145° C aus 2-Butanon/2-Propanol;

73 1-Benzyl-2-dimethylaminomethyl-5-octadecyloxy-4(1H)-pyridon . Oxalat . 1/2 $H_2O$, Schmp. 138-139° C aus 2-Propanol;

74 2-[N-(3-Dimethylaminopropyl)aminomethyl]-1-(2-methoxybenzyl)-5-octadecyloxy-4(1H)-pyridon . Difumarat, Schmp. 108-112° C aus 2-Propanol;

75 1-Benzyl-2-(N-benzyl-N-methylaminomethyl)-5-octadecyloxy-4(1H)-pyridon, Schmp. 59-61° C aus Ligroin;

76 2-[N-(3-Dimethylaminopropyl)aminomethyl]-1-(4-methylbenzyl)-5-octadecyloxy-4(1H)-pyridon . Trihydrochlorid . 1 2 $H_2O$, Schmp. 141-148° C aus Ethylacetat/2-Propanol;

77 2-[N-(4-Aminobutyl)aminomethyl]-1-benzyl-5-octadecyloxy-4(1H)-pyridon . Trihydrochlorid, Schmp. 179-183° C aus Ether Ethylacetat;

78 1-Benzyl-2-[N-(3-dimethylaminopropyl)aminomethyl]-5-(9-cis-octadecenyloxy)-4(1H)-pyridon • Trihydrochlorid • 1 2 $H_2O$, Schmp. 181-185° C aus Ethylacetat;

79 1-(4-Benzyloxyphenyl)-2-[N-(3-dimethylaminopropyl)aminomethyl]-5-octadecyloxy)-4(1H)-pyridon, Schmp. 43-47° C;

80 1-(4-Benzyloxybenzyl)-2-[N-(3-dimethylaminopropyl)aminomethyl]-5-octadecyloxy)-4(1H)-pyridon • Difumarat • 2,5 $H_2O$. Schmp. 103-109° C aus 2-Propanol/Ethylacetat;

81 1-Benzyl-2-[N-(3-dimethylaminopropyl)aminomethyl]-5-(2-octyldecyloxy)-4(1H)-pyridon • Trihydrochlorid • 3/4 $H_2O$, Schmp. 168-172° C aus Methylethylketon/2-Propanol;

82 5-(2-Adamantylethoxy)-1-benzyl-2-[N-(3-dimethylaminopropyl)aminomethyl]-4(1H)-pyridon, gelbes Öl, MS (m/l) 477 $M^+$;

83 (±)-1-Benzyl-5-octadecyloxy)-2-{N-[3-(1-phenylethyl)aminopropyl]aminomethyl}-4(1H)-pyridon • Trihydrochlorid, Schmp. 125-127° C mit Toluol verrührt;

## Beispiel 84

**2-[N-(3-Dimethylaminopropyl)aminomethyl]-1-(4-hydroxybenzyl)-5-octadecyloxy-4(1H)-pyridon • Dihydrochlorid • 2 $H_2O$**

Eine Lösung von 1,0 g (1,5 mMol) 1-(4-Benzyloxybenzyl)-2-[N-(3-dimethylaminopropyl)aminomethyl]-5-octadecyloxy-4(1H)-pyridon in 30 ml absolutem Ethanol wird mit 0,25 g Palladium auf Kohle (10%ig, Oxidform) versetzt und bei Raumtemperatur und Normaldruck hydriert. Nach Filtration vom Katalysator wird das Lösungsmittel abdestilliert und der Rückstand an Kieselgel mit Dichlormethan mit 3-6% Methanol mit $NH_3$-Gas gesättigt säulenchromatographiert. Das so erhaltene Reaktionsprodukt (wachsartige Substanz mit dem $R_f$ = 0,55, Kieselgel, Dichlormethan/Methanol mit $NH_3$ gesättigt 4:1) wird mit HCl-Gas-gesättigtem Methanol zur Salzbildung versetzt. Das Lösungsmittel wird abdestilliert und der Rückstand aus 2-Propalon/Ethylacetat umkristallisiert. Man erhält farblose Kristalle vom Schmp. 153-156° C.

In analoger Weise, wie in Beispiel 84 beschrieben, erhält man:

Beispiel Nr.

85 2-[N-(3-Dimethylaminopropyl)aminomethyl]-1-(4-hydroxyphenyl)-5-octadecyloxy-4(1H)-pyridon • Trihydrochlorid • 0,5 $H_2O$, Schmp. 236-237° C aus Methanol;

## Beispiel 86

**1-[(1-Benzyl-5-octadecyloxy-4(1H)-pyridon-2-yl)methyl]-4-cyano-4-phenylpiperidin . Dihydrochlorid**

Eine Lösung von 2,0 g (9 mMol) 4-Cyano-4-phenylpiperidin . Hydrochlorid in 40 ml Ethanol wird mit 4,8

ml (35 mMol) Triethylamin versetzt. Zu diesem Gemisch werden unter Rühren bei Raumtemperatur 5,0 g (8 mMol) 1-Benzyl-2-brommethyl-5-octadecyloxy-4(1H)-pyridon . Hydrobromid in Portionen eingetragen. Die Mischung wird 3 Stunden bei 60 °C gerührt, das Lösungsmittel im Vakuum abgezogen, der Rückstand mit Wasser und Dichlormethan versetzt, verrührt, die organische Phase abgetrennt, über $Na_2SO_4$ getrocknet und eingedampft. Der Rückstand wird zur Reinigung an Kieselgel mit Dichlormethan/Methanol 97:3 säulenchromatographiert. Man erhält ein öliges Produkt. 1,0 g (1,5 mMol) Substanz werden in 30 ml Ethylacetat gelöst und mit HCl-Gas-gesättigten Ether zur Salzbildung versetzt. Das ausgefallene Salz wird aus Ethylacetat/2-Propanol 6:1 umkristallisiert. Man erhält farblose Kristalle vom Schmp. 106- 112 °C, wobei der Feststoff etwa 3/4 Mol $H_2O$ pro Mol des genannten Produktes enthält.

In analoger Weise, wie in Beispiel 85 beschrieben, erhält man:

Beispiel Nr.

87 1-[(1-Benzyl-5-octadecyloxy-4(1H)-pyridon-2-yl)methyl]-4-methyloxycarbonyl-2-methylpiperazin . Dihydrochlorid . 3/4 $H_2O$, Schmp. 111-117 °C aus Ethylacetat/2-Propanol;

88 1-[(1-Benzyl-5-octadecyloxy-4(1H)-pyridon-2-yl)methyl]-4-benzylpiperazin, Schmp. 78-80 °C aus Diisopropylether;

89 1-Benzyl-2-[N-(3-dimethylaminopropyl)-N-methylaminomethyl]-5-octadecyloxy-4(1H)-pyridon . Trihydrochlorid, Schmp. 186-189 °C aus 2-Propanol/Ethanol ;

90 1-[(1-Benzyl-5-octadecyloxy-4(1H)-pyridon-2-yl)methyl]-4-diphenylmethylpiperazin • 1,5 Oxalat • $H_2O$, Schmp. 124-129 °C aus Ethylacetat;

91 1-[(1-Benzyl-5-octadecyloxy-4(1H)-pyridon-2-yl)methyl]-4-phenylpiperazin • Dihydrochlorid • $H_2O$, Schmp. 80 °C (sintern) aus Ethylacetat/Methanol;

92 1-[(1-Benzyl-5-octadecyloxy-4(1H)-pyridon-2-yl)methyl]-4-(2-pyrimidinyl)piperazin, Schmp. 96-97 °C aus Diisopropylether/2-Propanol;

93 1-[(1-Benzyl-5-octadecyloxy-4(1H)-pyridon-2-yl)methyl]-4-benzylpiperazin • Dioxalat • 1/2 $H_2O$, Schmp. 133-136 °C aus Ethanol;

94 1-[(1-Benzyl-5-octadecyloxy-4(1H)-pyridon-2-yl)methyl]-4-(2-phenylethyl)piperazin • Trihydrochlorid • 1/2 $H_2O$, Schmp. 161-164 °C aus Ethylacetat/2-Propanol;

95 1-[(1-Benzyl-5-decyloxy-4(1H)-pyridon-2-yl)methyl]-4-(2-phenylethyl)piperazin • Dihydrochlorid, Schmp. 161-164 °C aus Ethylacetat/2-Propanol;

96 1-[(1-Benzyl-5-benzyloxy-4(1H)-pyridon-2-yl)methyl]-4-benzylpiperazin • Trihydrochlorid, Schmp. 189-191 °C aus Ethylacetat/2-Propanol;

97 (±)-1-[(1-Benzyl-5-octadecyloxy-4(1H)-pyridon-2-yl)methyl]-4-(1-phenylethyl)piperazin • Trihydrochlorid 3/4 $H_2O$, Schmp. 167-170 °C aus Acetonitril/2-Propanol;

98 (±)-1-[(5-Octadecyloxy-1-(1-phenylethyl)-4(1H)-pyridon-2-yl)methyl]-4-benzylpiperazin, Schmp. 76-78 °C aus Diisopropylether;

99 (-)-1-[(5-Octadecyloxy-1-(1-phenylethyl)-4(1H)-pyridon-2-yl)methyl]-4-benzylpiperazin, Schmp. 60-61 °C aus Diisopropylether, $[\alpha]_D$ = -2,3 ° (C = 2,06 Methanol);

100 (+)-1-[(5-Octadecyloxy-1-(1-phenylethyl)-4(1H)-pyridon-2-yl)methyl]-4-benzylpiperazin, Schmp. 60-61 °C aus Diisopropylether, $[\alpha]_D$ = +2,4 ° (C = 2,01 Methanol);

Beispiel 101

1-[(1-Benzyl-5-octadecyloxy-4(1H)-pyridon-2-yl)methyl]-4-aminomethyl-4-phenylpiperidin . Trihydrochlorid

2,7 g (4,1 mMol) 1-[(1-Benzyl-5-octadecyloxy-4(1H)-pyridon-2-yl)methyl]-4-cyano-4-phenylpiperidin werden mit 60 ml $NH_3$-Gas-gesättigtem Methanol und 1 g Raney Nickel versetzt und bei 60 °C und 60 bar im Autoklaven mit Wasserstoff hydriert. Nach 6 Stunden ist die Reaktion beendet. Nach Abfiltration des Katalysators wird die hellgelbe Lösung im Vakuum eingedampft und der Rückstand an Kieselgel mit Dichlormethan/Methanol, mit $NH_3$ gesättigt, 97:3, säulenchromatographiert. Das so erhaltene Reaktionsprodukt (ölige Substanz mit dem Rf = 0,5; Kieselgel, Dichlormethan/ Methanol mit $NH_3$ gesätt., 9:1) wird in Ethylacetat gelöst und mit HCl-Gas-gesättigtem Ether zur Salzbildung versetzt. Das ausgefallene Salz wird aus 2-Butanon/2-Propanol 10:1 umkristallisiert. Man erhält farblose Kristalle vom Schmp. 192-197 °C. Der Feststoff enthält etwa 1,25 Mol Wasser pro Mol des genannten Produktes.

Beispiel 102

(+/-)-1-[(1-Benzyl-5-octadecyloxy-4(1H)-pyridon-2-yl)methyl]-2-methylpiperazin . Fumarat

Eine Mischung aus 2 g (3,2 mMol) (+/-)-1-[(1-Benzyl-5-octadecyloxy-4(1H)-pyridon-2-yl)methyl]-4-methoxycarbonyl-2-methylpiperazin, 5 ml 33% HBr/Essigsäure und 5 ml Essigsäure wird 2 Stunden im geschlossenen Gefäß gerührt. Nach Abziehen der Hauptmenge des Lösungsmittel im Vakuum wird der Rückstand mit Wasser versetzt, mit 2N Natronlauge alkalisiert, mit Dichlormethan extrahiert, die organische Phase abgetrennt, über $Na_2SO_4$ getrocknet und eingedampft. Das erhaltene Harz wird zur Reinigung an Kieselgel mit Dichlormethan/Methanol, mit $NH_3$-Gas-gesättigt, 97:3 säulenchromatographiert. Das dabei erhaltene Reaktionsprodukt (ölige Substanz mit dem Rf = 0,2; Kieselgel, Dichlormethan/Methanol mit $NH_3$-Gas-gesättigt 9:1) wird in 2-Propanol gelöst und mit einer Lösung von Fumarsäure in 2-Propanol versetzt. Nach 24 Stunden Stehen wird die klare Lösung eingedampft und das Salz aus 2-Butanon umkristallisiert. Man erhält farblose Kristalle vom Schmp. 151-155 °C. Der Feststoff enthält etwa 1 Mol $H_2O$ pro Mol des genannten Produktes.

Beispiel 103

1-Benzyl-2-(3-dimethylaminopropyloxymethyl)-5-octadecyloxy-4(1H)-pyridon . Dihydrochlorid

7,2 g (15 mMol) 1-Benzyl-2-hydroxymethyl-5-octadecyloxy-4(1H)-pyridon werden in 110 ml absol. Dimethylformamid bei 70 °C gelöst und portionsweise mit 0,60 g (20 mMol) Natriumhydrid (80% in Paraffinöl) versetzt. Nach 1,5 Stunden Nachrühren bei 70-80 °C wird eine Lösung von 3,3 g (27,15 mMol) 3-Dimethylaminopropylchlorid in 20 ml absol. Dimethylformamid zugetropft. Die Reaktionsmischung läßt man 2 Stunden bei 70 °C rühren. Danach dampft man die Lösung im Vakuum zur Trockene ein und versetzt den Rückstand mit Wasser und Dichlormethan. Die organische Phase wird abgetrennt, über $Na_2SO_4$ getrocknet und eingedampft. Der Rückstand wird an Kieselgel mit Dichlormethan/ Methanol mit $NH_3$-Gas gesättigt 97:3 säulenchromatographiert. Man erhält eine ölige Substanz. 1,8 g (3,3 mMol) Substanz werden in ein Gemisch aus 30 ml Ethylacetat und 3,5 ml 2-Propanol gelöst und mit HCl-Gas gesättigten Ether zur Salzbildung versetzt. Das ausgefallene Salz wird aus Ethylacetat/ 2-Propanol 3:1 umkristallisiert. Man erhält farblose Kristalle vom Schmp. 155-159 °C. Der Feststoff enthält etwa 1/4 Mol Wasser pro Mol des genannten Produktes.

In analoger Weise, wie in Beispiel 103 beschrieben, erhält man:

Beispiel Nr.

104 2-(3-Dimethylaminopropyloxymethyl)-1-methyl-5-octadecyloxy-4(1H)-pyridon . Dihydrochlorid, Schmp. 210-216 °C aus 2-Propanol;

105 2-(3-Dimethylaminopropyloxymethyl)-5-octadecyloxy-1-propyl-4(1H)-pyridon . Dihydrochlorid . 3/4 $H_2O$, Schmp. 127-128 °C aus Ethylacetat/2-Propanol;

106 2-(3-Dimethylaminopropyloxymethyl)-1-(4-methylbenzyl)-5-octadecyloxy-4(1H)-pyridon . Dihydrochlorid . 1,5 $H_2O$, Schmp. 78-84 °C aus Ethylacetat/2-Propanol;

107 2-(3-Dimethylaminopropyloxymethyl)-1-(4-methoxybenzyl)-5-octadecyloxy-4(1H)-pyridon . Dihydrochlorid . 1/4 $H_2O$, Schmp. 144-147 °C aus 2-Propanol;

108 1-Cyclohexylmethyl-2-(3-dimethylaminopropyloxymethyl)-5-octadecyloxy-4(1H)-pyridon . Oxalat, Schmp. 81-84 °C aus Ethylacetat/2-Propanol;

109 1-(4-Chlorbenzyl)-2-(3-dimethylaminopropyloxymethyl)-5-octadecyloxy-4(1H)-pyridon . Dihydrochlorid . 2,5 $H_2O$, Schmp. 70-74 °C aus Ethylacetat/2-Propanol);

110 2-(3-Dimethylaminopropyloxymethyl)-1-(2-methoxybenzyl)-5-octadecyloxy-4(1H)-pyridon . 1,5 Oxalat, Schmp. 89-81 °C aus Ethylacetat/2-Propanol;

Beispiel 111

3-[1-Benzyl-5-octadecyloxy-4(1H)-pyridon-2-yl]-propionsäurenitril . Hydrochlorid

A. 3-[1-Benzyl-5-octadecyloxy-4(1H)-pyridon-2-yl]-2-cyanpropionsäure-tert.-butylester

Zur Lösung von 17,0 g (0,15 Mol) Kalium-tert.-butylat in 60 ml absol. Dimethylformamid wird unter Rühren bei Raumtemperatur die Lösung von 21,2 g (0,15 Mol) Cyanessigsäure-tert.-butylester in 15 ml absol. Dimethylformamid getropft. Die klare Lösung wird auf 50° C erwärmt und bei dieser Temperatur eine Lösung von 43,4 g (0,07 Mol) 1-Benzyl-2-brommethyl-5-octadecyloxy-4(1H)-pyridon . Hydrobromid in 150 ml absol. Dimethylformamid in 5 Minuten zugegeben. Nach 1 Stunde Rühren bei 55° C wird das Reaktionsgemisch zu 1,2 l Wasser gegeben und mit Ethylacetat extrahiert. Die organische Phase wird abgetrennt, über $Na_2SO_4$ getrocknet und eingeengt. Der ölige Rückstand wird zur Reinigung an Kieselgel mit Dichlormethan/Methanol 99:1 säulenchromatographiert (Rf = 0,60; Kieselgel, Dichlormethan/Methanol 9:1). Man erhält ein gelbes, öliges Produkt.

B. 3-[1-Benzyl-5-octadecyloxy-4(1H)-pyridon-2-yl]-2-cyanpropionsäure

13,2 g (22 mMol) 3-[1-Benzyl-5-octadecyloxy-4(1H)-pyridon-2-yl]- 2-cyan-propionsäure-tert.-butylester werden mit 47 ml 0,5 N Kaliumhydroxid in methanol versetzt und 3 Tage bei Raumtemperatur gerührt. Das Lösungsmittel wird im Vakuum abgezogen, der Rückstand in Wasser gelöst und mit 2 N Salzsäure angesäuert, der ausgefallene Niederschlag abgesaugt, mit Wasser und Ether gewaschen und getrocknet. Man erhält ein fast farbloses Produkt vom Schmp. 122-124° C, das für die weitere Umsetzung genügend rein ist.

C. 3-[1-Benzyl-5-octadecyloxy-4(1H)-pyridon-2-yl]-propionsäurenitril . Hydrochlorid

4.1 g (7,4 mMol) 3-[1-Benzyl-5-octadecyloxy-4(1H)-pyridon-2-yl]- 2-cyan-propionsäure werden 30 Minuten bei 150° C erhitzt. Nach dem Abkühlen erhält man das Produkt als Öl, das für die weitere Umsetzung genügend rein ist. 0,5 g (1 mMol) Substanz werden in Ethylacetat gelöst und mit HCl-Gas gesättigtem Ether zur Salzbildung versetzt. Das ausgefallene Salz wird abgesaugt und getrocknet. Man erhält ein beiges Produkt vom Schmp. 98-102° C.

In analoger Weise, wie in Beispiel 111 beschrieben, erhält man:

Beispiel Nr.:

112 3-(1-Methyl-5-octadecyloxy-4(1H)-pyridon-2-yl)-propionsäurenitril, Schmp. 85-87° C mit Ether digeriert;

Beispiel 113

3-[1-Benzyl-5-octadecyloxy-4(1H)-pyridon-2-yl]propylamin • Dihydrochlorid

3,7 g (7,3 mMol) 3-[1-Benzyl-5-octadecyloxy-4(1H)-pyridon-2-yl]-propionsäurenitril werden mit 40 ml mit $NH_3$-Gas-gesättigtem Methanol versetzt und bei 60° C und 60 bar im Autoklaven mit Wasserstoff hydriert. Nach Filtration vom Katalysator wird die Lösung im Vakuum eingedampft und der ölige Rückstand an Kieselgel mit Dichlormethan/Methanol, mit $NH_3$ gesättigt, 95:5, säulenchromatographiert. So erhält man 2,2 g (59 % d. Th.) ölige Substanz (Rf = 0,1; Kieselgel, Dichlormethan/Methanol mit $NH_3$ gesättigt 9:1), die mit etherischer Salzsäure in das Hydrochlorid übergeführt und aus 2-Butanon/2-Propanol 4:1 umkristallisiert wird. Man erhält farblose Kristalle vom Schmp. 138-143° C.

Beispiel 114

3-[ 1-Benzyl-5-octadecyloxy-4(1H)-pyridon-2-yl]propionsäuremethylester

3,0 g (6 mMol) 3-[1-Benzyl-5-octadecyloxy-4(1H)-pyridon-2-yl]propionsäurenitril werden in 30 ml mit HCl-Gas-gesättigtem Methanol gelöst und 3 Tage bei Raumtemperatur im verschlossenen Gefäß stehen gelassen. Nach Abziehen des Lösungsmittels im Vakuum wird der Rückstand mit Wasser versetzt, mit verd. Natriumhydroxidlösung alkalisiert und mit Dichlormethan extrahiert. Die Dichlormethanlösung wird über $Na_2SO_4$ getrocknet und eingedampft. Das erhaltene Wachs wird zweimal aus Diisopropylether umkristallisiert. Man erhält farblose Kristalle vom Schmp. 78-81 °C.

Beispiel 115 und 116

3-[1-Benzyl-5-octadecyloxy-4(1H)-pyridon-2-yl]propanol und 3-[1-Benzyl-5-octadecyloxy-4(1H)-pyridon-2-yl]-propionsäureamid

5,0 g (10 mMol) 3-[1-Benzyl-5-octadecyloxy-4(1H)-pyridon-2-yl]propionsäurenitril in 30 ml 2-Propanol werden unter Rühren bei Raumtemperatur mit 2,5 g (6,6 mMol) Natriumborhydrid versetzt und das Gemisch 4 Tage bei 80 °C weitergerührt. Nach Abziehen des Lösungsmittels im Vakuum wird der Rückstand mit verd. Natriumhydroxidlösung versetzt und 30 Minuten bei 40 °C gerührt. Nach Abkühlen wird mit Dichlormethan extrahiert, die organische Phase mit Wasser gewaschen, getrocknet und eingedampft. Der Rückstand wird an Kieselgel mit Dichlormethan/Methanol säulenchromatographiert. Durch Elution mit Dichlormethan mit 1-5 % Methanol wird der rohe 3-[1-Benzyl-5-octadecyloxy-4(1H)-pyridon-2-yl]propanol erhalten (Rf = 0.30: Kieselgel, Dichlormethan/Methanol 9:1). Nach Umkristallisation aus Diisopropylether/ 2-Propanol 4:1 erhält man hellbeige Kristalle vom Schmp. 87-89 °C.

Durch weitere Elution der Säule mit Dichlormethan mit 6-10% Methanol wird das rohe 3-[1-Benzyl-5-octadecyloxy-4(1H)-pyridon-2-yl]-propionsäureamid erhalten (Rf = 0,40; Kieselgel, Dichlormethan/Methanol 9:1). Nach Umkristallisation aus 2-Butanon erhält man farblose Kristalle vom Schmp. 134-136 °C.

Hemmung der Aktivität von Proteinkinase C

Zur Klärung des hemmenden Einflusses der erfindungsgemäßen Verbindungen der allgemeinen Formel I auf die Calcium- und Phospholipid-abhängige Proteinkinase (PKC) wurde diese Enzymaktivität aus Rattenhirn angereichert. In Anlehnung an in der Literatur beschriebene Reinigungsschritte (J. Biol. Chem. 260, 15718-15722, 1985) wurde bei der aus 2 Schritten bestehenden Reinigung die Eigenschaft des Enzyms ausgenutzt, in Anwesenheit von Calcium an Zellmembranen zu binden und durch Calciumchelatoren (EGTA) wieder abgelöst zu werden. Beim 1. Anreicherungsschritt erfolgte die Bindung der PKC an Membranen des Rattenhirns und im 2. Schritt an sogenannte Inside/Out-Vesikel von Erythrozyten. Nach Ablösung von den Erythrozyten-Membranen und Umpufferung lag die PKC-Präparation in 10mM HEPES, 1 mM DTT, 0,1% PEG 20000, pH 7,5 vor und konnte unter diesen Bedingungen bei -70 °C für mehrere Monate ohne Aktivitätsverlust gelagert werden.

Die Aktivität des Enzyms wurde über den Einbau von Phosphor-32-markiertem Phosphat in das durch PKC phosphorylierbare Protein Histon H-1 (Sigma Typ III) bestimmt.

Der Test enthält dabei folgende Komponenten:

50 mM HEPES-NaOH (pH 7,5), 5mM $MgCl_2$, 1 mM DTT, 4 µM freies $Ca^2$, 10 µM ATP, 1 µg Phosphatidylserin, 0,2 µg 1,2-Diolein sowie 40µg Histon H-1 und gegebenenfalls die Testsubstanz.

Der Ansatz wird 4 min bei 30 °C vorinkubiert und die Reaktion dann durch Zusatz von 5 nM PKC gestartet. Nach 5 min Inkubation bei 30 °C wird die Reaktion mit 10% TCA gestoppt und die Proben dann abfiltriert. Der Phosphateinbau wird mittels Cerenkov-Zählung im Scintillationszähler bestimmt. Die in Abwesenheit der Testsubstanz gemessene Kinase-Aktivität wurde jeweils als 100% gesetzt und die Hemmwirkung der Verbindung der allgemeinen Formel I prozentual darauf bezogen (Tabelle I).

Endothelabhängige Relaxation der Gefäßmuskulatur

Zur Anwendung kommen Gefäßringe der Kaninchenaorta, die zwischen zwei L-förmigen Haken im Organbad fixiert werden. Die Präparate befinden sich in einer physiologischen Salzlösung (nach Krebs-Henseleit), die von Carbogen durchperlt wird. Die Kontraktionen werden durch 3 x $10^{-7}$ mol/l Noradrenalin ausgelöst. Wenn die Spannung ein stabiles Plateau erreicht hat, erfolgt die Zugabe der Testsubstanzen. Die Substanzwirkung wird zuerst in Präparaten mit intaktem Endothel und anschließend, um die Endothelabhängigkeit der gefundenen Effekte zu überprüfen, in Präparaten mit zerstörtem Endothel getestet. Für eine endothelabhängige Relaxation, die durch den vom Endothel stammenden relaxierenden Faktor (EDRF, endothelium-derived relaxing factor) bewirkt wird, gelten die Kriterien: Abwesenheit des Effektes in Präparaten mit zerstörtem Endothel, keine Hemmbarkeit der Relaxation durch Indometazin und Hemmbarkeit durch

NDGA (Nordihydro-guaiaretsäure), Methylenblau und Gossypol. Diese Kriterien werden von den in der Tabelle II angeführten Verbindungen voll erfüllt, so daß gefolgert werden kann, daß die angeführten Verbindungen unerwarteterweise über die Freisetzung des EDRF wirken.

Tabelle I

| Beispiel Nr. | Hemmung der Proteinkinase C ($IC_{50}$; Mol/ml) |
|---|---|
| 19 | $2,4 \times 10^{-6}$ |
| 54 | $1,4 \times 10^{-6}$ |
| 56 | $2,9 \times 10^{-6}$ |
| 57 | $1,9 \times 10^{-6}$ |
| 59 | $2,9 \times 10^{-6}$ |
| 60 | $2,7 \times 10^{-6}$ |
| 61 | $2,1 \times 10^{-6}$ |
| 65 | $6,5 \times 10^{-6}$ |
| 66 | $2,5 \times 10^{-6}$ |
| 68 | $1,9 \times 10^{-6}$ |
| 69 | $1,5 \times 10^{-6}$ |
| 70 | $2,2 \times 10^{-6}$ |
| 71 | $1,8 \times 10^{-6}$ |
| 72 | $3,6 \times 10^{-6}$ |
| 73 | $4,4 \times 10^{-6}$ |
| 74 | $2,1 \times 10^{-6}$ |
| 75 | $9,5 \times 10^{-6}$ |
| 76 | $1,9 \times 10^{-6}$ |
| 77 | $2,3 \times 10^{-6}$ |
| 78 | $4,6 \times 10^{-6}$ |
| 79 | $2,2 \times 10^{-6}$ |
| 80 | $2,6 \times 10^{-6}$ |
| 83 | $2,6 \times 10^{-6}$ |
| 84 | $3,4 \times 10^{-6}$ |
| 85 | $5,8 \times 10^{-6}$ |
| 88 | $3,0 \times 10^{-6}$ |
| 89 | $1,8 \times 10^{-6}$ |
| 93 | $5,5 \times 10^{-6}$ |
| 94 | $2,4 \times 10^{-6}$ |

| | |
|---|---|
| 95 | $4,3 \times 10^{-6}$ |
| 97 | $4,4 \times 10^{-6}$ |
| 98 | $5,4 \times 10^{-6}$ |
| 99 | $5,2 \times 10^{-6}$ |
| 100 | $2,7 \times 10^{-6}$ |
| 101 | $1,5 \times 10^{-6}$ |
| 102 | $2,0 \times 10^{-6}$ |
| 103 | $4,0 \times 10^{-6}$ |
| 105 | $2,0 \times 10^{-6}$ |
| 106 | $2,4 \times 10^{-6}$ |
| 107 | $2,9 \times 10^{-6}$ |
| 108 | $2,9 \times 10^{-6}$ |
| 109 | $2,6 \times 10^{-6}$ |
| 114 | $9,0 \times 10^{-6}$ |
| 116 | $9,0 \times 10^{-6}$ |

Tabelle II

| Beispiel Nr. | Relaxation der Noradrenalin ($3 \times 10^{-7}$ mol/l) vorkontrahierten Aorta; $EC_{50}$, mol/l | |
|---|---|---|
| | Endothel intakt | Endothel entfernt |
| 54 | $1,5 \times 10^{-5}$ | $> 10^{-4}$ |
| 101 | $2 \times 10^{-5}$ | $> 10^{-4}$ |
| 69 | $3 \times 10^{-5}$ | $> 10^{-4}$ |
| 71 | $3 \times 10^{-5}$ | $> 10^{-4}$ |
| 88 | $3,4 \times 10^{-5}$ | $> 10^{-4}$ |
| Acetylcholin | $3 \times 10^{-8}$ | $> 10^{-4}$ |

## Ansprüche

1. Alkoxy-4(1H)-pyridon-Derivate der allgemeinen Formel I

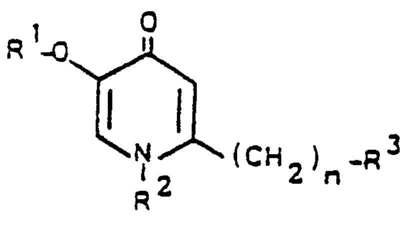

(I)

in welcher

$R^1$ eine geradkettige oder verzweigte, gesättigte oder ungesättigte Alkyl- oder Adamantylalkylgruppe mit jeweils bis zu 22 Kohlenstoffatomen darstellt,

$R^2$ eine geradkettige oder verzweigte, gesättigte oder ungesättigte Alkylgruppe mit bis zu 5 Kohlenstoffatomen, eine Cycloalkylmethylgruppe mit 5 bis 7 C-Atomen im Cycloalkylring, eine unsubstituierte oder durch Halogen, Hydroxy, Alkyl mit bit zu 5 C-Atomen, Alkoxy mit bis zu 5 C-Atomen, Dialkylamino mit bis zu 5 C-Atomen oder Benzyloxy substituierte Phenyl- oder Phenylalkylgruppe mit einem geradkettigen oder verzweigten Alkylrest mit bis zu 5 C-Atomen oder eine Aminoalkylgruppe der allgemeinen Formel II darstellt

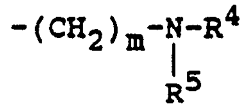

(II)

worin $R^4$ und $R^5$ gleich oder verschieden sind und Wasserstoff oder eine Alkylgruppe mit 1 bis 3 C-Atomen und m eine ganze Zahl von 2 bis 5 bedeuten,

n eine ganze Zahl von 1 bis 5 bedeutet, und

$R^3$ entweder

a) ein Halogenatom, eine Hydroxy-, Cyano-, Carboxamid- oder Alkoxycarbonylgruppe mit bis zu 5 C-Atomen in der Alkylgruppe bedeutet, oder einen Aminorest der allgemeinen Formel III

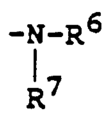

(III)

bedeutet, worin $R^6$ und $R^7$ gleich oder verschieden sind und Wasserstoff, eine Alkyl- oder omega-Hydroxyalkylgruppe mit bis zu 5 C-Atomen, eine unsubstituierte oder durch Halogen, Hydroxy, Alkyl mit bis zu 5 C-Atomen, Alkoxy mit bis zu 5 C-Atomen, Dialkylamino mit bis zu 5 C-Atomen oder Benzyloxy substituierte Phenyl- oder Phenylalkylgruppe mit einem geradkettigen oder verzweigten Alkylrest mit bis zu 5 C-Atomen bedeuten, oder

b) einen Aminorest der allgemeinen Formel IV

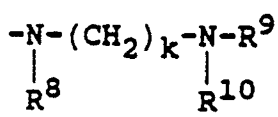

(IV)

darstellt, in welcher $R^8$ Wasserstoff oder eine Alkylgruppe mit bis zu 5 C-Atomen und $R^9$ und $R^{10}$ gleich oder verschieden sind und Wasserstoff, eine Alkylgruppe mit bis zu 5 C-Atomen, eine unsubstituierte oder durch Halogen, Hydroxy, Alkyl mit bis zu 5 C-Atomen, Alkoxy mit bis zu 5 C-Atomen, Dialkylamino mit bis zu 5 C-Atomen oder Benzyloxy substituierte Phenyl- oder Phenylalkylgruppe mit einem geradkettigen oder verzweigten Alkylrest mit bis zu 5 C-Atomen bedeuten und k eine ganze Zahl von 2 bis 5 bedeutet, oder

c) einen Heterocyclus der allgemeinen Formel V

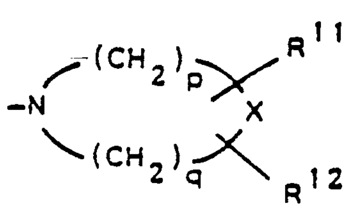

(V)

bedeutet, in welcher $R^{11}$ und $R^{12}$ gleich oder verschieden sind und Wasserstoff, eine Alkylgruppe mit bis zu 3 C- Atomen oder eine Phenyl- oder Phenylalkylgruppe mit einem geradkettigen oder verzweigten Alkylrest mit bis zu 5 C-Atomen bedeuten, X für Sauerstoff, einen Aminorest der allgemeinen Formel VI,

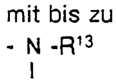 (VI)

wobei $R^{13}$ Wasserstoff oder eine Phenyl-, Phenylalkyl-oder Diphenylalkylgruppe mit einem geradkettigen oder verzweigten Alkylrest mit bis zu 5 C-Atomen, wobei im Phenylring bis zu 3 CH-Gruppen durch Stickstoffatome ersetzt sein können, bedeutet, oder für einen Rest der allgemeinen Formel VII

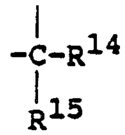

(VII)

steht, worin $R^{14}$ Wasserstoff oder eine unsubstituierte oder durch Halogen, Hydroxy, Alkyl mit bis zu 5 C-Atomen, Alkoxy mit bis zu 5 C-Atomen, Dialkylamino mit bis zu 5 C-Atomen oder Benzyloxy substituierte Phenylgruppe bedeutet, $R^{15}$ Wasserstoff, eine Hydroxy-, Hydroxymethyl-, Cyano-, Aminomethyl-, Carboxamid-, Ethoxy- oder Methoxycarbonylgruppe und p und q gleich oder verschieden sein können und 2 oder 3 bedeuten, oder

d) für einen Rest der allgemeinen Formel VIII

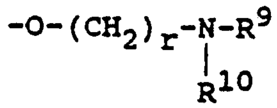

(VIII)

steht, worin $R^9$ und $R^{10}$ die oben genannte Bedeutung haben, und r eine ganze Zahl von 2 bis 5 bedeutet, sowie deren pharmakologisch unbedenkliche Salze.

2. Verbindungen der allgemeinen Formel I gemäß Anspruch 1, dadurch gekennzeichnet, daß

$R^1$ eine geradkettige oder verzweigte, gesättigte oder ungesättigte Alkyl- oder Adamantylalkylgruppe mit jeweils 2 bis 22 Kohlenstoffatomen,

$R^2$ eine geradkettigen oder verzweigten Alkylrest mit 1 bis 4 C-Atomen, einen Cyclohexylmethylrest, einen unsubstituierten oder durch Halogen, Hydroxy, Methyl, Benzyloxy, Methoxy oder Dimethylamino monosubstituierten Phenyl- oder Phenylalkylgruppe mit einem geradkettigen oder verzweigten Alkylrest mit bis zu 5 C-Atomen bedeutet,

n eine ganze Zahl von 1 bis 3 bedeutet, und

$R^3$ entweder,

a) ein Halogenatom, eine Hydroxy-, Cyano-, Carboxamid- oder Alkoxycarbonylgruppe mit bis zu 5 C-Atomen in der Alkylgruppe oder einen Rest der allgemeinen Formel III darstellt, in welcher $R^6$ und $R^7$ gleich oder verschieden sind und Wasserstoff, eine Methyl-, omega-Hydroxypropyl-oder Benzylgruppe bedeuten, oder

b) einen Aminorest der allgemeinen Formel IV darstellt, in welcher $R^8$ Wasserstoff oder eine Methylgruppe bedeutet, $R^9$ und $R^{10}$ gleich oder verschieden sind und Wasserstoff, eine Methyl-, Benzyl- oder Phenylethylgruppe und k, 2, 3 oder 4 bedeuten, oder

c) einen Heterocyclus der allgemeinen Formel V darstellt, in welcher $R^{11}$ und $R^{12}$ gleich oder verschieden sind und Wasserstoff oder eine Methylgruppe bedeuten, X für einen Aminorest der allgemeinen Formel VI steht, wobei $R^{13}$ Wasserstoff oder eine Phenyl-, Phenylalkyl- oder Diphenylalkylgruppe mit einem geradkettigen oder verzweigten Alkylrest mit bis zu 5 C-Atomen, wobei im Phenylring bis zu 3 CH-Gruppen durch Stickstoffatome ersetzt sein können, bedeutet, oder X für einen Rest der allgemeinen Formel VII steht, worin $R^{14}$ für eine Phenylgruppe, $R^{15}$ eine Cyano- oder Aminomethylgruppe bedeutet, und p und q gleich 2 sind, oder

d) einen Rest der allgemeinen Formel VIII darstellt, in welcher $R^9$ und $R^{10}$ Methylgruppen bedeuten und r gleich 2 oder 3 ist.

3. Verbindungen der allgemeinen Formel I gemäß Anspruch 1 oder 2, dadurch gekennzeichnet, daß

n gleich 1, 2 oder 3 ist,

$R^1$ einen Butyl-, Decyl-, Tetradecyl-, Eicosanyl-, Octadecyl-, Octadecenyl-, 2-Octyldecyl- oder Adamantylethylrest,

$R^2$ einen Propyl-, Methyl-, Phenyl-, Benzyl-, Methylbenzyl, Chlorbenzyl-, Methoxybenzyl-, Cyclohexylmethyl-, Dimethylaminobenzyl-, Dimethylaminopropyl-, Hydroxybenzyl-, Benzyloxybenzyl-, Benzyloxyphenyl-, Hydroxyphenyl- oder Phenylethylrest und

$R^3$ einen Hydroxy-, Brom-, Cyano-, Amino-, Carboxamid-, Methoxycarbonyl-, Dimethylaminopropylamino-,

Dimethylaminopropyl-N-methylamino-, Hydroxypropylamino-, Dimethylaminoethyl-N-methylamino-, Aminopropylamino-, Aminoethylamino-, Aminobutylamino-, Dimethylamino-, Dimethylaminopropyloxy-, N-Benzyl-N-methylaminopropylamino-, N-Benzyl-N-methylamino-, Methyloxycarbonyl-methylpiperazin-, Benzylpiperazin-, Diphenylmethylpiperazin-, Aminomethylphenylpiperidin-, Phenylpiperazin-, Methylpiperazin-, Phenylethylpiperazin- oder Pyrimidinylpiperazinrest bedeutet.

4. Verfahren zur Herstellung von Alkoxy-4(1H)-pyridon-Derivaten der allgemeinen Formel I gemäß Anspruch 1 bis 4, dadurch gekennzeichnet, daß man entweder

a) eine Verbindung der allgemeinen Formel IX

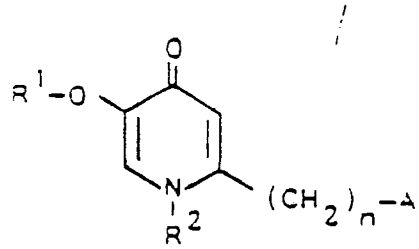

(IX)

in welcher $R^1$, $R^2$ die oben angegebene Bedeutung haben, n gleich 1 ist und A für eine nukleofuge Abgangsgruppe steht, mit Aminoverbindungen der allgemeinen Formel X, XI und XII,

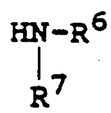 ,

(X)

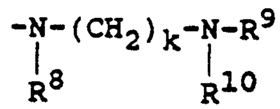 ,

(XI)

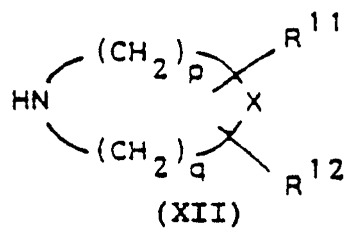

(XII)

in welcher $R^6$, $R^7$, $R^8$, $R^9$, $R^{10}$, $R^{11}$, $R^{12}$, k, p, q und x die oben angegebene Bedeutung besitzen, in an sich bekannter Weise umsetzt, oder

b) eine Verbindung der allgemeinen Formel XIII,

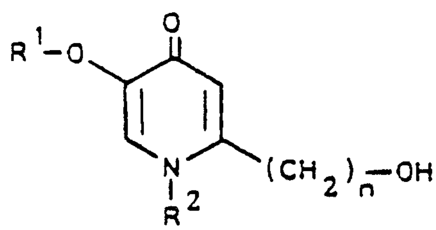

(XIII)

in welcher $R^1$, $R^2$ die oben angegebene Bedeutung haben und n gleich 1 ist, mit einer Verbindung der allgemeinen Formel XIV,

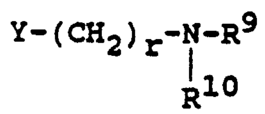

(XIV)

in welcher $R^9$, $R^{10}$ und r die oben genannte Bedeutung haben und Y eine Abgangsgruppe bedeutet, in einem geeigneten Lösungsmittel unter basischen Bedingungen, umsetzt, oder

c) wenn n gleich 2 oder 3 ist und $R^3$ für eine Amino-, Hydroxy-, Cyano-, Methoxycarbonyl- oder Carboxamidgruppe steht, ein 2-Halogenmethylpyridon der allgemeinen Formel IXa

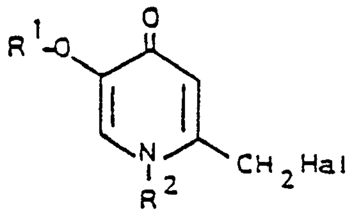

IXa

in welcher $R^1$ und $R^2$ die oben angegebene Bedeutung haben, mit einem Cyanessigsäureester nach an sich bekannter Weise umsetzt, verseift und decarboxyliert und aus den gemäß a), b) oder c) erhaltenen Cyanoverbindungen der allgemeinen Formel Ia

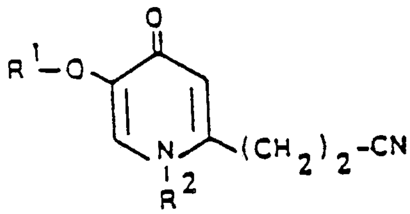

Ia

nach literaturbekannten Methoden die Ester-, Amino-, Hydroxy- und Amidoverbindungen der allgemeinen Formel I herstellt.

5. Arzneimittel enthaltend neben üblichen Hilfs- und Zusatzstoffen Verbindungen der allgemeinen Formel I gemäß Anspruch 1.

6. Verwendung von Verbindungen der allgemeinen Formel I gemäß Anspruch 1 zur Prävention und/oder Behandlung von Herz- und Gefäßkrankheiten wie Thrombosen, Arteriosklerose, Hypertonien, von Entzündungsprozessen, Allergien, Krebs und bestimmten degenerativen Schäden des Zentralnervensystems.

7. Verwendung von Verbindungen der allgemeinen Formel I gemäß Anspruch 1 zur Herstellung von Pharmazeutika zur Prävention und/oder Behandlung von Herz- und Gefäßkrankheiten wie Thrombosen, Arteriosklerose, Hypertonien, von Entzündungsprozessen, Allergien, Krebs und bestimmten degenerativen Schäden des Zentralnervensystems.

Europäisches Patentamt

# EUROPÄISCHER TEILRECHERCHENBERICHT,
der nach Regel 45 des Europäischen Patent-übereinkommens für das weitere Verfahren als europäischer Recherchenbericht gilt

Nummer der Anmeldung

EP 89 11 4477

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X,D | BULLETIN OF THE CHEMICAL SOCIETY OF JAPAN, Band 52, Nr. 1, Januar 1979, Seiten 107-110; K. IMAFUKU et al.: "Structure of 5-hydroxy-2-hydroxymethyl-4-pyridones" | | C 07 D 213/69<br>A 61 K 31/44<br>C 07 D 401/06<br>C 07 D 401/14<br>A 61 K 31/495 |
| | * Seite 107, Verbindung VI * | 1,2 | |
| | -- | | |
| X | US-A-2 965 641 (C.P. KRIMMEL) | | |
| | * Spalte 1, Zeilen 59-66; Spalte 2, Zeilen 5-10; Beispiele 1,2,4, 6,9 * | 1,2,4, 5 | |
| | -- | | |
| X,D | J. HETEROCYCLIC CHEM., Band 23, Nr. 1, Januar-Februar 1986; J.H. LOOKER et al.: "Convenient preparative methods for N-aryl-gamma-pyridones from gamma-pyrones" | | RECHERCHIERTE SACHGEBIETE (Int. Cl.4) |
| | * Verbindungen 2-8 * ./. | 1,2 | C 07 D 213/00<br>C 07 D 401/00<br>A 61 K 31/00 |

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-5,7

Unvollständig recherchierte Patentansprüche:

Nicht recherchierte Patentansprüche: 6

Grund für die Beschränkung der Recherche:

Verfahren zur chirurgischen oder therapeutischen Behandlung des menschlischen oder tierischen Körpers (Siehe Art. 52(4) des Europäischen Patentübereinkommens).

| Recherchenort | Abschlußdatum der Recherche | Prüfer |
|---|---|---|
| Den Haag | 16-10-1989 | DE JONG |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

Europäisches Patentamt

# EUROPÄISCHER TEILRECHERCHENBERICHT

Nummer der Anmeldung
EP 89 11 4477



| EINSCHLÄGIGE DOKUMENTE | | | KLASSIFIKATION DER ANMELDUNG (Int. Cl.4) |
|---|---|---|---|
| Kategorie | Kennzeichnung des Dokuments mit Angabe, soweit erforderlich, der maßgeblichen Teile | betrifft Anspruch | |
| A | EP-A-0 120 670 (NATIONAL RESEARCH DEVELOPMENT CORP.)<br>* Anspruch 1 *<br>----- | 1,5 | |
| | | | RECHERCHIERTE SACHGEBIETE (Int. Cl.4) |